# EUROPEAN PATENT APPLICATION

(11) **EP 4 692 119 A1**
(43) Date of publication of application: **11.02.2026**
(21) Application number: 24778080.2
(22) Date of filing: 27.03.2024
(51) Int. Cl.: C07K 16/28, C12N 15/13, C12N 15/85, C12N 5/10, A61K 39/395, A61P 35/00, A61P 37/02, A61P 43/00

(54) **ANTIBODY OR ANTIGEN-BINDING FRAGMENT THEREOF TARGETING CD3 AND USE THEREOF**

(30) Priority: 28.03.2023 CN 202310316471
(71) Applicant: Sanyou Biopharmaceuticals Co., Ltd., Shanghai 201114 (CN)
(72) Inventor: XU, Peifang, Shanghai 201114 (CN); LANG, Guojun, Shanghai 201114 (CN); LIU, Chanjuan, Shanghai 201114 (CN); WANG, Liyan, Shanghai 201114 (CN); LI, Shuangqi, Shanghai 201114 (CN); YAN, Xintian, Shanghai 201114 (CN); FANG, Xiaopeng, Shanghai 201114 (CN); HU, Yuhao, Shanghai 201114 (CN); SUN, Xinglu, Shanghai 201114 (CN); GU, Jiahui, Shanghai 201114 (CN); HAN, Yangyang, Shanghai 201114 (CN); ZHU, Yiling, Shanghai 201114 (CN); SHEN, Fei, Shanghai 201114 (CN)
(74) Representative: Latscha Schöllhorn Partner AG
(86) International application number: PCT/CN2024/084158
(87) International publication number: WO 2024/199294

(57) **Abstract**

Provided is an anti-CD3 antibody or antigen-binding fragment thereof specifically binding to human and non-human primate CD3. The anti-CD3 antibody or antigen-binding fragment thereof has increased or decreased affinity and can achieve the purpose of increasing drug efficacy or reducing toxicity. When forming a bispecific antibody, selective adaptation can be performed according to different targets, providing a flexible adaptation scheme for drug development. In addition, provided are a conjugate, fusion, bispecific antibody, or pharmaceutical composition of the anti-CD3 antibody or antigen-binding fragment thereof. Further provided are a nucleic acid encoding the antibody, a host cell comprising the nucleic acid, a method for preparing the antibody, and a use of the foregoing CD3-binding antibody in preventing or treating cancer, an infectious disease, or an autoimmune disease in a subject.

## Description

### Technical Field

The present invention relates to the field of antibody drugs. Specifically, the present invention relates to an anti-CD3 antibody that specifically binds to human and non-human primate CD3 and activates T cells with reduced activation activity, as well as a conjugate, fusion, or bispecific antibody comprising the anti-CD3 antibody or antigen-binding fragment thereof. Furthermore, the present invention relates to a nucleic acid encoding the anti-CD3 antibody and a host cell comprising the nucleic acid, as well as a method for producing the antibody. The present invention further relates to a pharmaceutical composition comprising the anti-CD3 antibody and to the medical use of the anti-CD3 antibody.

### Background Art

The body's immune system is composed of central lymphoid organs, peripheral lymphoid organs, immune cells and immune molecules. The process of immune response depends on the interaction between cells in the immune system, including direct contact between cells and cell-cell interaction through the release of cytokines or other mediators. Immune cells, especially T cells, play a core role in the entire process of immune response. There are many CD molecules on the surface of T cells, such as CD3, CD4, CD8, CD28, etc. These molecules are extensively involved in the entire process of immune effects, ranging from T cell recognition of antigens, activation and proliferation or anergy, apoptosis, clearance of alloantigens, to establishment of tolerance toward self-antigens. Therefore, people try to regulate the function and state of T cells by using monoclonal antibodies to specifically bind to CD molecules, thus improving the body's immunity.

Among these CD molecules, the CD3 molecule is particularly important. The CD3 molecule is a protein complex that is usually expressed on the surface of mature T cells. It has four peptide chains: γ, δ, ε, and ζ, which are composed of three parts: the extracellular region, the transmembrane region, and the cytoplasmic region. The N-terminal extracellular domain contains two heterodimeric domains: CD3εγ and CD3εδ. The transmembrane domain is a single homodimer CD3ζζ, which forms a TCR-CD3 complex with the α/β chain of the T cell receptor (TCR) through a salt bridge by a non-covalent bond, and the complex has the function of activating the signal transduction cascade in T cells. Many therapeutic strategies modulate T cell immunity by targeting TCR signaling, particularly anti-human CD3 monoclonal antibodies (mAbs) that are widely used clinically in immunosuppressive regimens.

The specific mouse anti-human CD3 monoclonal antibody OKT3 is the first monoclonal antibody approved for use in humans. This antibody recognizes the ε chain of the CD3 molecule (Sgro, Toxicology 105 (1995), 23-29), and is widely used clinically as an immunosuppressant (Chatenoud, Clin. Transplant 7 (1993)), 422-430; Chatenoud, Nat. Rev. Immunol. 3 (2003), 123-132; Kumar, Transplant. Proc.30 (1998), 1351-1352). It has been reported in the literature that OKT3 can effectively promote T cell mitosis (Van Wauve, J. Immunol. 124 (1980), 2708-2718) and induce T cell killing function (Wong, Transplantation 50 (1990), 683-689). OKT3 demonstrates both activities of stimulating or blocking T cells at different timelines. OKT3 activates T cells in the early stage of T cells and causes cytokine release. Further administration of OKT3 will block all known T cell functions. It is because of the blockade of T cell function in the late stage by OKT3 that OKT3 has gained wide application in immunotherapy, such as being used as an immunosuppressant to reduce or eliminate allogeneic organ transplantation rejection.

In addition to being an immunosuppressant, CD3 antibody can also be used as a T cell agonist to fight the immune escape of tumor cells, which is also the key to the current large-scale use of CD3 bispecific antibodies in the development of anti-tumor drugs. However, studies have found that many CD3 antibodies are species-specific, and they only recognize a single site or epitope on the target CD3 molecule. For example, OKT-3 reacts only with chimpanzee CD3 but not with the CD3 homologue of macaques, which are also primates. The anti-CD3 monoclonal antibody UCHT-1 also reacts with chimpanzee CD3 but not with macaque CD3. The monoclonal antibody FN-18 recognizes only the macaque CD3 antigen but does not recognize the human CD3 counterpart (Uda et al., J. Med. Primatol. 30 (2001), 141-147).

KLINGER et al. discovered a CD3 monoclonal antibody H2C (WO 2008119567 A2) that can simultaneously bind to human and non-chimpanzee primate CD3ε chains, exhibiting cross-species specific binding domains, which makes the antibody useful in preclinical animal studies as well as human clinical studies. Studies have shown that the epitope to which monoclonal antibody H2C binds is a polypeptide fragment of N-terminal amino acid residues 1-27 of the extracellular domain of CD3ε. This epitope is different from all other known epitopes of CD3ε described in the art in that it maintains its three-dimensional structure.

At present, more than 100 bispecific antibodies targeting CD3+ T cells are in the clinical research stage. Although bispecific antibodies have made great achievements in safety and effectiveness, they also face many challenges. For example, excessive activation of CD3+ T cells by anti-CD3 antibodies, which are components of bispecific antibodies, would lead to abnormal cytokine secretion, thus inducing cytokine syndrome, resulting in high toxic and side effects of CD3 bispecific antibodies.

Accordingly, there is an urgent need in the art for anti-CD3 antibodies with diverse functional properties, which can provide enhanced efficacy or reduced toxicity when the antibodies are used to form bispecific antibodies.

### Summary of the Invention

Through studies, the present inventors have obtained a group of cross-species anti-CD3 antibodies that activate T cells with reduced activating activity, having one or more of the following properties:
(i) a monomer purity greater than 90%, preferably greater than 92%, 94%, 96%, 97%, as measured by SEC-HPLC;
(ii) a purity greater than 90%, preferably greater than 91%, 92%, or 93%, as measured by reducing or non-reducing SDS-PAGE;
(iii) specifically binding to CD3 expressed on a cell surface.

In a first aspect, the present invention provides an antibody or antigen-binding fragment that specifically binds to CD3, comprising
(a) 3 CDRs in a heavy chain variable region amino acid sequence as set forth in SEQ ID NO: 28 and 3 CDRs in a light chain variable region amino acid sequence as set forth in SEQ ID NO: 29;
(b) 3 CDRs in a heavy chain variable region amino acid sequence as set forth in SEQ ID NO: 1 and 3 CDRs in a light chain variable region amino acid sequence as set forth in SEQ ID NO: 31;
(c) 3 CDRs in a heavy chain variable region amino acid sequence as set forth in SEQ ID NO: 32 and 3 CDRs in a light chain variable region amino acid sequence as set forth in SEQ ID NO: 33;
(d) 3 CDRs in a heavy chain variable region amino acid sequence as set forth in SEQ ID NO: 1 and 3 CDRs in a light chain variable region amino acid sequence as set forth in SEQ ID NO: 34;
(e) 3 CDRs in a heavy chain variable region amino acid sequence as set forth in SEQ ID NO: 28 and 3 CDRs in a light chain variable region amino acid sequence as set forth in SEQ ID NO: 36;
(f) 3 CDRs in a heavy chain variable region amino acid sequence as set forth in SEQ ID NO: 39 and 3 CDRs in a light chain variable region amino acid sequence as set forth in SEQ ID NO: 40; or
(g) 3 CDRs in a heavy chain variable region amino acid sequence as set forth in SEQ ID NO: 28 and 3 CDRs in a light chain variable region amino acid sequence as set forth in SEQ ID NO: 43.

In some embodiments, the antibody or antigen-binding fragment of the present invention that specifically binds to CD3 comprises a heavy chain variable region and a light chain variable region, wherein
(a) the heavy chain variable region comprises, according to AbM numbering, HCDR1 as set forth in GFTFSKYAMN (SEQ ID NO: 51); HCDR2 as set forth in RIRSKYNNYATY (SEQ ID NO: 46); and HCDR3 as set forth in HGNFGNSYISYWAY (SEQ ID NO: 47); the light chain variable region comprises, according to AbM numbering, LCDR1 as set forth in GSSTGAVTSGYYPN (SEQ ID NO: 48); LCDR2 as set forth in GTKFLAP (SEQ ID NO: 49) ; and LCDR3 as set forth in ALWYSNRWV (SEQ ID NO: 50);
(b) the heavy chain variable region comprises, according to AbM numbering, HCDR1 as set forth in GFTFNKYAMN (SEQ ID NO: 45); HCDR2 as set forth in RIRSKYNNYATY (SEQ ID NO: 46); and HCDR3 as set forth in HGNFGNSYISYWAY (SEQ ID NO: 47); the light chain variable region comprises, according to AbM numbering, LCDR1 as set forth in GSSTGAVTSGYYPN (SEQ ID NO: 48); LCDR2 as set forth in GTKFLAP (SEQ ID NO: 49); and LCDR3 as set forth in ALWVDNRWV (SEQ ID NO: 55);
(c) the heavy chain variable region comprises, according to AbM numbering, HCDR1 as set forth in GFTFPKYAMN (SEQ ID NO: 52); HCDR2 as set forth in RIRSKYNNYETY (SEQ ID NO: 53); and HCDR3 as set forth in HGNFGNSYISYWAY (SEQ ID NO: 47); the light chain variable region comprises, according to AbM numbering, LCDR1 as set forth in GSSTGAVTSGYYPN (SEQ ID NO: 48); LCDR2 as set forth in GTHFLAP (SEQ ID NO: 54) ; and LCDR3 as set forth in ALWYSNRWV (SEQ ID NO: 50);
(d) the heavy chain variable region comprises, according to AbM numbering, HCDR1 as set forth in GFTFNKYAMN (SEQ ID NO: 45); HCDR2 as set forth in RIRSKYNNYATY (SEQ ID NO: 46); and HCDR3 as set forth in HGNFGNSYISYWAY (SEQ ID NO: 47); the light chain variable region comprises, according to AbM numbering, LCDR1 as set forth in GSSTGAVTSGYYPN (SEQ ID NO: 48); LCDR2 as set forth in GTKFLAP (SEQ ID NO: 49); and LCDR3 as set forth in ALWQENRWV (SEQ ID NO: 56);
(e) the heavy chain variable region comprises, according to AbM numbering, HCDR1 as set forth in GFTFSKYAMN (SEQ ID NO: 51); HCDR2 as set forth in RIRSKYNNYATY (SEQ ID NO: 46); and HCDR3 as set forth in HGNFGNSYISYWAY (SEQ ID NO: 47); the light chain variable region comprises, according to AbM numbering, LCDR1 as set forth in GSSTGAVTSGYYPN (SEQ ID NO: 48); LCDR2 as set forth in GTKFLAP (SEQ ID NO: 49); and LCDR3 as set forth in ALWVDNRWV (SEQ ID NO: 55);
(f) the heavy chain variable region comprises, according to AbM numbering, HCDR1 as set forth in GFTFSKYAMN (SEQ ID NO: 51); HCDR2 as set forth in RIRSKYNNYETY (SEQ ID NO: 53); and HCDR3 as set forth in HGNFGNSYISYWAY (SEQ ID NO: 47); the light chain variable region comprises, according to AbM numbering, LCDR1 as set forth in GSSTGAVTSGYYPN (SEQ ID NO: 48); LCDR2 as set forth in GTHFLAP (SEQ ID NO: 54); and LCDR3 as set forth in ALWYSNRWV (SEQ ID NO: 50); or
(g) the heavy chain variable region comprises, according to AbM numbering, HCDR1 as set forth in GFTFSKYAMN (SEQ ID NO: 51); HCDR2 as set forth in RIRSKYNNYATY (SEQ ID NO: 46); and HCDR3 as set forth in HGNFGNSYISYWAY (SEQ ID NO: 47); the light chain variable region comprises, according to AbM numbering, LCDR1 as set forth in GSSTGAVTSGYYPN (SEQ ID NO: 48); LCDR2 as set forth in GTKFLAP (SEQ ID NO: 49); and LCDR3 as set forth in ALWQENRWV (SEQ ID NO: 56).

In some embodiments, the antibody or antigen-binding fragment of the present invention that specifically binds to CD3 comprises a heavy chain variable region and a light chain variable region, wherein the antibody or antigen-binding fragment comprises
(a) a heavy chain variable region as set forth in SEQ ID NO: 26 and a light chain variable region as set forth in SEQ ID NO: 27;
(b) a heavy chain variable region as set forth in SEQ ID NO: 28 and a light chain variable region as set forth in SEQ ID NO: 29;
(c) a heavy chain variable region as set forth in SEQ ID NO: 30 and a light chain variable region as set forth in SEQ ID NO: 2;
(d) a heavy chain variable region as set forth in SEQ ID NO: 1 and a light chain variable region as set forth in SEQ ID NO: 31;
(e) a heavy chain variable region as set forth in SEQ ID NO: 32 and a light chain variable region as set forth in SEQ ID NO: 33;
(f) a heavy chain variable region as set forth in SEQ ID NO: 1 and a light chain variable region as set forth in SEQ ID NO: 34;
(g) a heavy chain variable region as set forth in SEQ ID NO: 30 and a light chain variable region as set forth in SEQ ID NO: 27;
(h) a heavy chain variable region as set forth in SEQ ID NO: 35 and a light chain variable region as set forth in SEQ ID NO: 29;
(i) a heavy chain variable region as set forth in SEQ ID NO: 30 and a light chain variable region as set forth in SEQ ID NO: 29;
(j) a heavy chain variable region as set forth in SEQ ID NO: 28 and a light chain variable region as set forth in SEQ ID NO: 36;
(k) a heavy chain variable region as set forth in SEQ ID NO: 30 and a light chain variable region as set forth in SEQ ID NO: 31;
(l) a heavy chain variable region as set forth in SEQ ID NO: 37 and a light chain variable region as set forth in SEQ ID NO: 38;
(m) a heavy chain variable region as set forth in SEQ ID NO: 39 and a light chain variable region as set forth in SEQ ID NO: 40;
(n) a heavy chain variable region as set forth in SEQ ID NO: 41 and a light chain variable region as set forth in SEQ ID NO: 33;
(o) a heavy chain variable region as set forth in SEQ ID NO: 26 and a light chain variable region as set forth in SEQ ID NO: 42;
(p) a heavy chain variable region as set forth in SEQ ID NO: 28 and a light chain variable region as set forth in SEQ ID NO: 43; or
(q) a heavy chain variable region as set forth in SEQ ID NO: 30 and a light chain variable region as set forth in SEQ ID NO: 44.

In some embodiments, the antibody or antigen-binding fragment of the present invention that specifically binds to CD3 is an IgG1, IgG2, IgG3, or IgG4 antibody; optionally, an IgG1 or IgG4 antibody; optionally, an IgG1 antibody. In some embodiments, the antigen-binding fragment is Fab, Fab', F(ab')₂, Fv, single chain Fv, single chain Fab or diabody.

In a second aspect, the present invention provides a nucleic acid encoding the antibody of the first aspect of the present invention, a vector comprising the nucleic acid encoding the antibody, a host cell comprising the nucleic acid molecule or vector, and a method for preparing the antibody. The method comprises culturing, under conditions suitable for the expression of a nucleic acid encoding an antibody or antigen-binding fragment that specifically binds to a CD3 molecule of the first aspect of the present invention, a host cell into which an expression vector comprising the nucleic acid encoding the antibody or antigen-binding fragment that specifically binds to the CD3 molecule of the first aspect of the present invention has been introduced. Optionally, the method further comprises recovering the antibody or antigen-binding fragment that specifically binds to the CD3 molecule from the host cell culture. Preferably, the host cell is prokaryotic or eukaryotic, more preferably selected from E. coli cells, yeast cells, mammalian cells or other cells suitable for preparing antibodies or antigen-binding fragments thereof, most preferably, the host cell is a HEK293 cell or a CHO cell.

In a third aspect, the present invention relates to a conjugate, fusion or bispecific antibody comprising the anti-CD3 antibody or antigen-binding fragment thereof of the first aspect of the present invention.

In a fourth aspect, the present invention relates to a pharmaceutical composition comprising the anti-CD3 antibody or antigen-binding fragment thereof of the first aspect of the present invention, or the conjugate, fusion or bispecific antibody of the third aspect of the present invention, and optionally a pharmaceutically acceptable carrier.

In a fifth aspect, the present invention relates to the use of the anti-CD3 antibody or antigen-binding fragment thereof of the first aspect of the present invention, the conjugate, fusion or bispecific antibody of the third aspect of the present invention, or the pharmaceutical composition of the fourth aspect of the present invention for preparing a drug for preventing or treating cancer, an infectious disease or an autoimmune disease in a subject.

The present invention has the beneficial effects that: anti-CD3 antibodies with different affinities are provided to achieve the purpose of increasing drug efficacy or reducing toxicity; when forming a bispecific antibody, selective adaptation can be performed according to different targets, providing a flexible adaptation scheme for drug development.

### Brief Description of the Drawings

The following detailed description of the preferred embodiments of the present invention can be better understood when reading in conjunction with the following drawings below. For the purpose of illustrating the present invention, there are shown in the drawings embodiments which are presently preferred. However, it should be understood that the present invention is not limited to the precise arrangements and instrumentalities of the embodiments shown in the drawings.

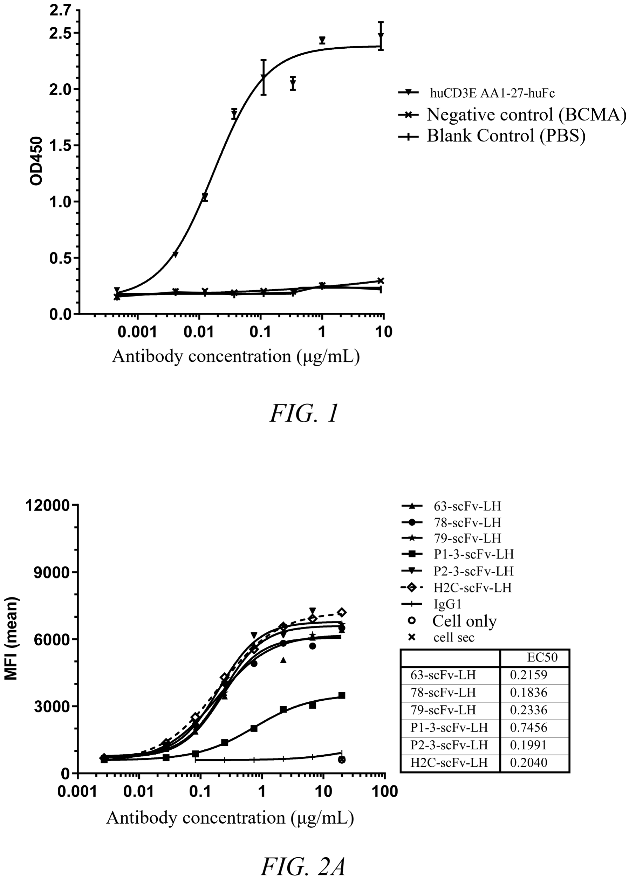

FIG. 1 shows that antibody H2C (IgG1) binds to the antigen huCD3E-AA1-27-huFc constructed and expressed in example 1.

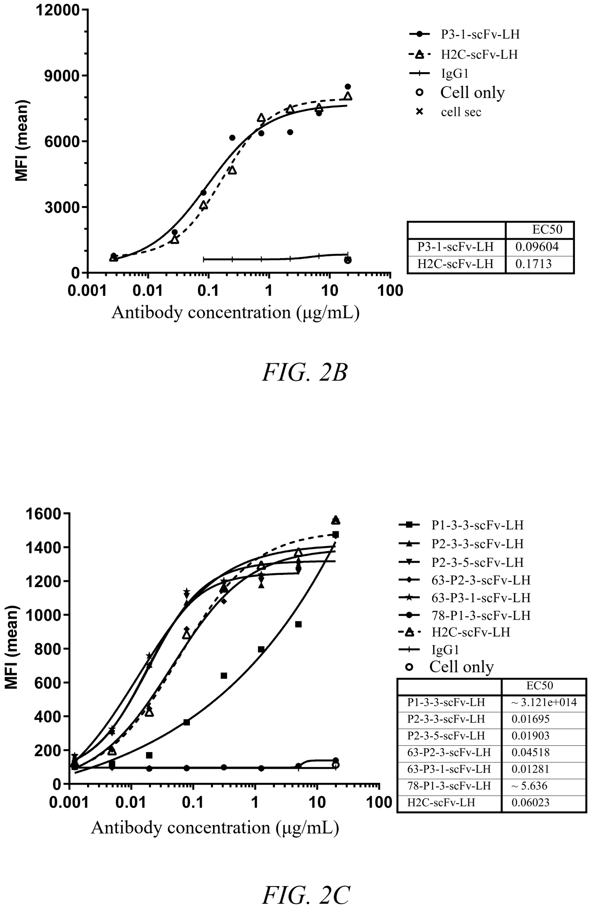

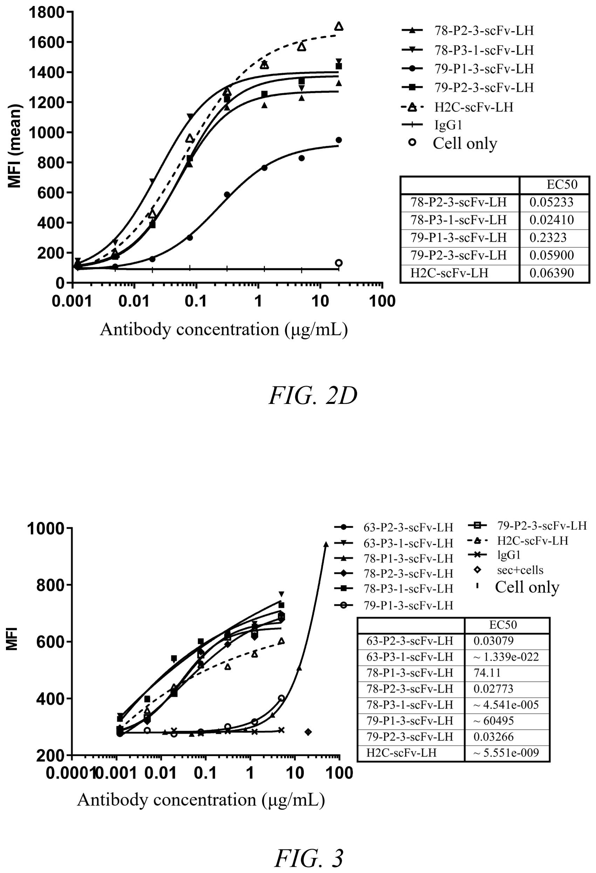

FIGs. 2A-2D show the results of antibodies binding to huCD3D-CD3E-CHO-K1 cells or Jurkat cells as detected by FACS. FIG. 2A shows the results of binding of P1-3-scFv-LH, P2-3-scFv-LH, 63-scFv-LH, 78-scFv-LH, 79-scFv-LH to huCD3D-CD3E-CHO-K1 cells. FIG. 2B shows the results of binding of P3-1-scFv-LH to huCD3D-CD3E-CHO-K1 cells. FIG. 2C shows the results of binding of P1-3-3-scFv-LH, P2-3-3-scFv-LH, P2-3-5-scFv-LH, 63-P2-3-scFv-LH, 63-P3-1-scFv-LH, and 78-P1-3-scFv-LH to Jurkat cells. FIG. 2D shows the results of binding of 78-P2-3-scFv-LH, 78-P3-1-scFv-LH, 79-P1-3-scFv-LH, and 79-P2-3-scFv-LH to Jurkat cells.
FIG. 3 shows the results of detection of binding of antibodies 63-P2-3-scFv-LH, 63-P3-1-scFv-LH, 78-P1-3-scFv-LH, 78-P2-3-scFv-LH, 78-P3-1-scFv-LH, 79-P1-3-scFv-LH, 79-P2-3-scFv-LH to CynoCD3E-HEK293 cells based on FACS.

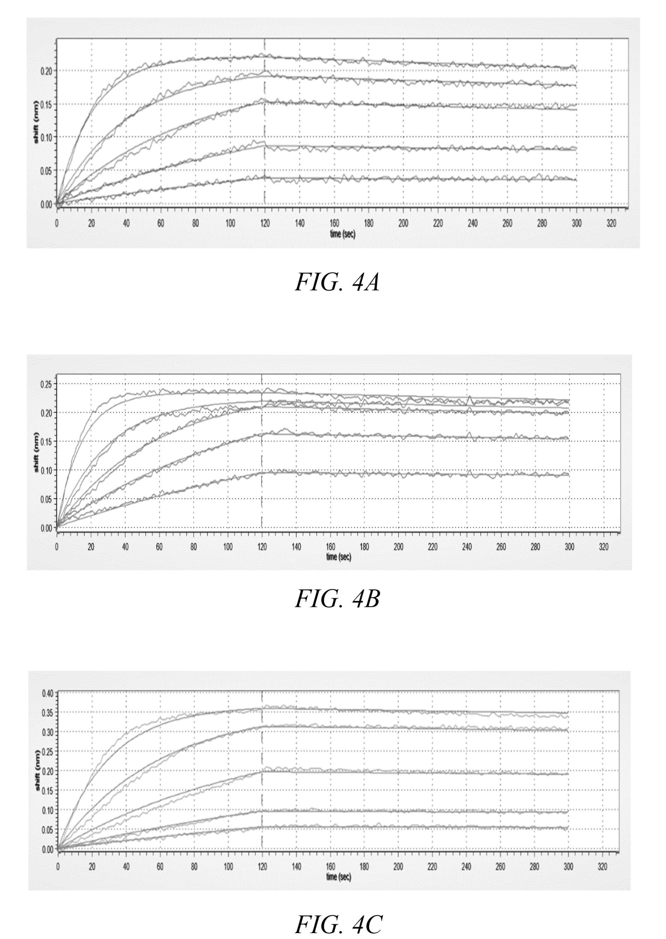

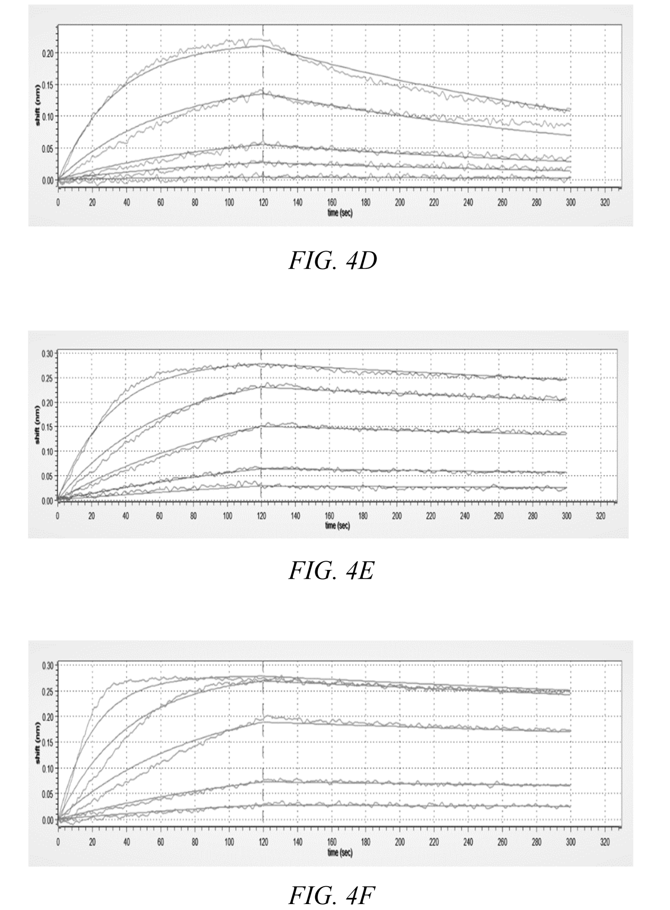

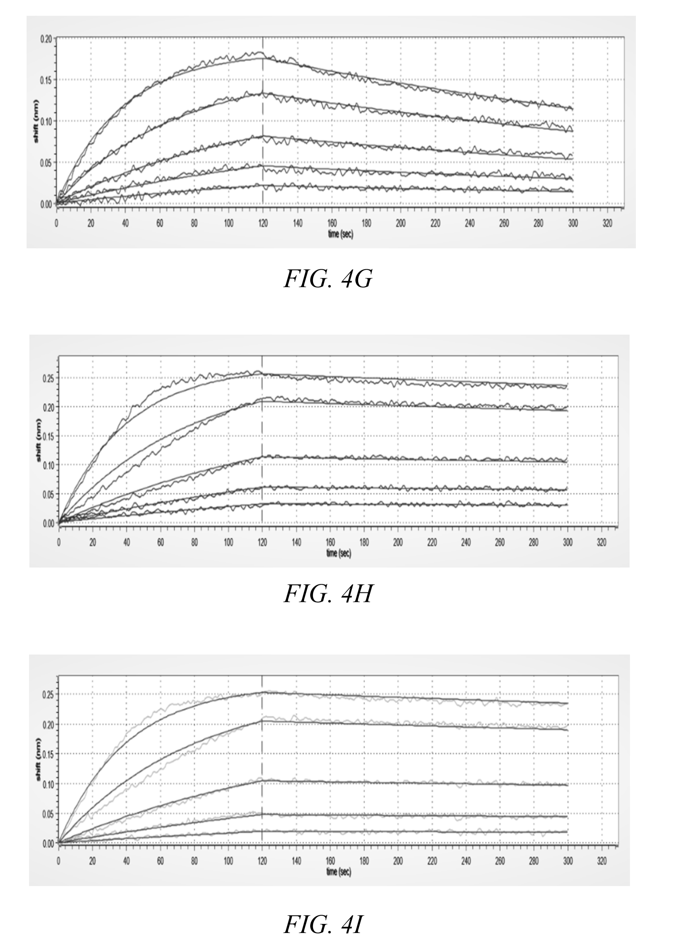

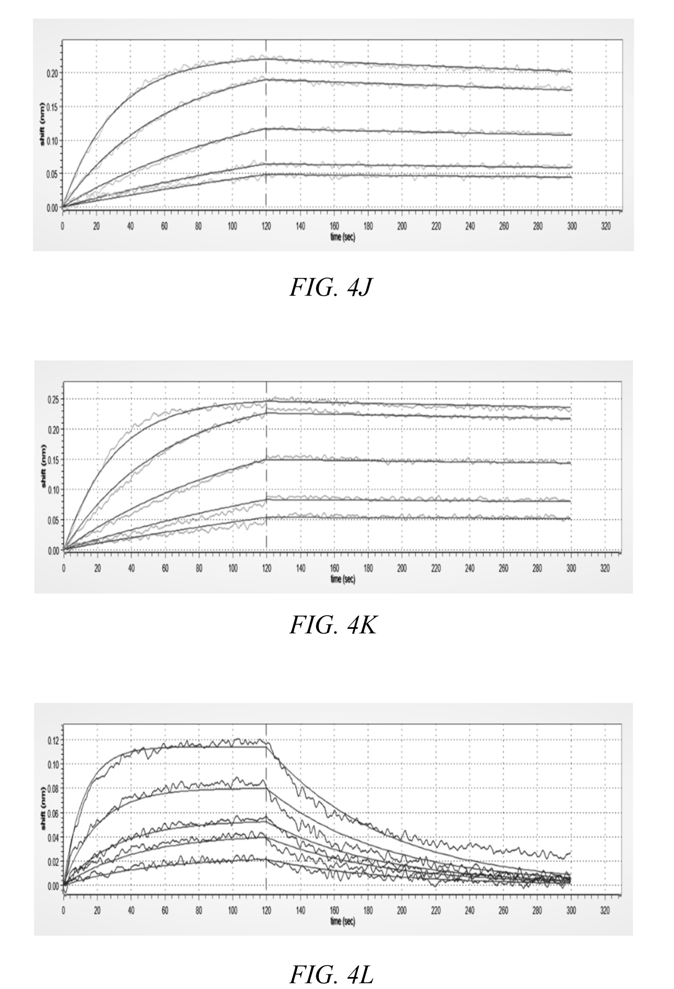

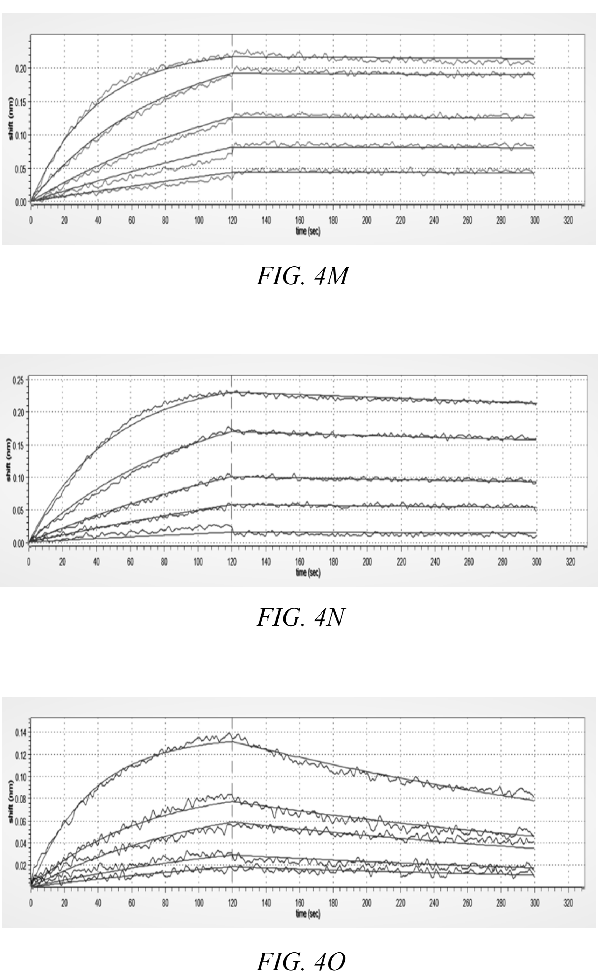

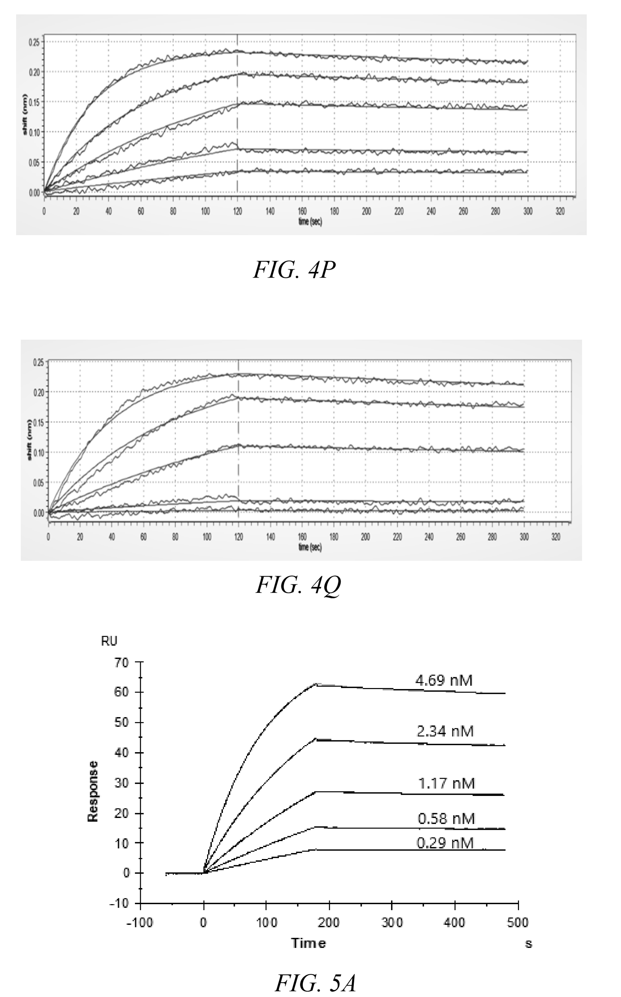

FIGs. 4A-4Q show the results of detection of affinity of 63-scFv-LH, 78-scFv-LH, 79-scFv-LH, P1-3-scFv-LH, P2-3-scFv-LH, P3-1-scFv-LH, P1-3-3-scFv-LH, P2-3-3-scFv-LH, P2-3-5-scFv-LH, 63-P2-3-scFv-LH, 63-P3-1-scFv-LH, 78-P1-3-scFv-LH, 78-P2-3-scFv-LH, 78-P3-1-scFv-LH, 79-P1-3-scFv-LH, 79-P2-3-scFv-LH and 79-P3-1-scFv-LH to huCD3E-AA1-27-huFc antigen protein based on BLI, respectively.

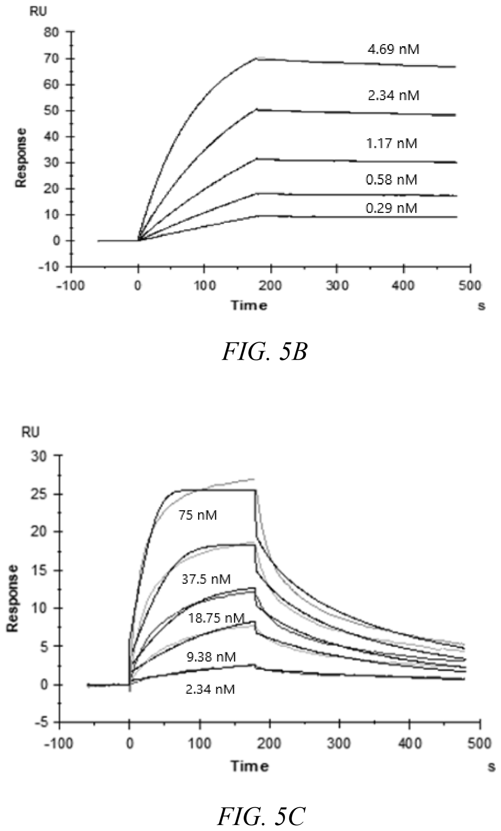

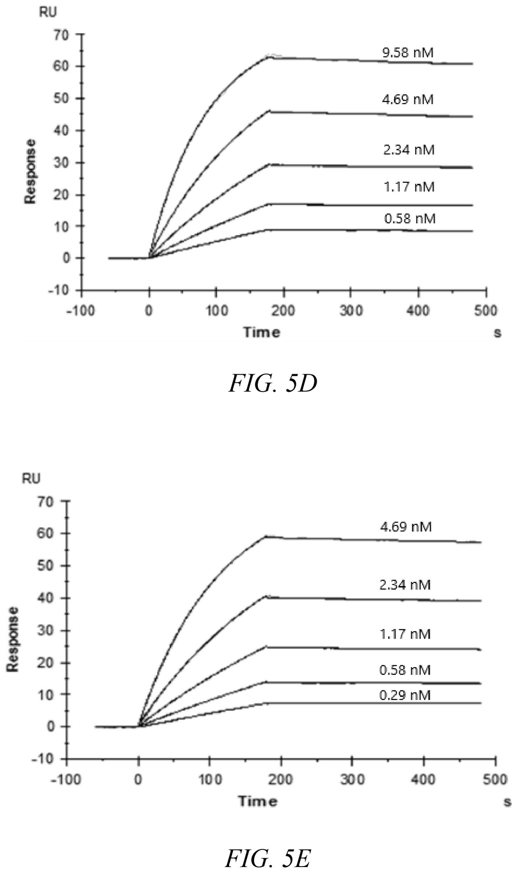

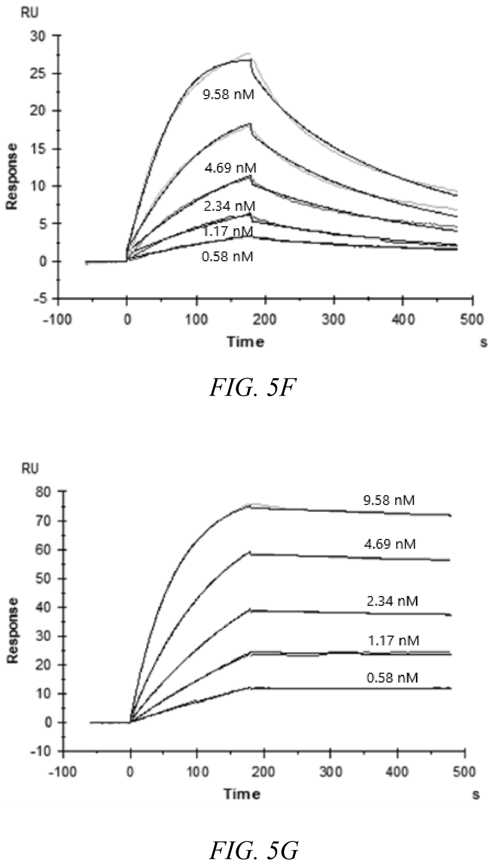

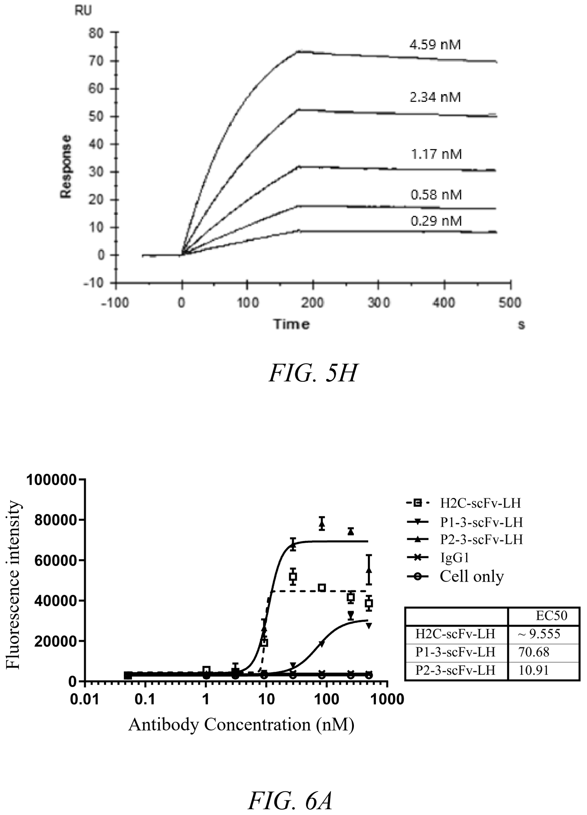

FIGs. 5A-5H show the results of detection of affinity of 63-P2-3-scFv-LH, 63-P3-1-scFv-LH, 78-P1-3-scFv-LH, 78-P2-3-scFv-LH, 78-P3-1-scFv-LH, 79-P1-3-scFv-LH, 79-P2-3-scFv-LH and H2C-scFv-LH to huCD3E-AA1-27-huFc antigen protein based on SPR, respectively.

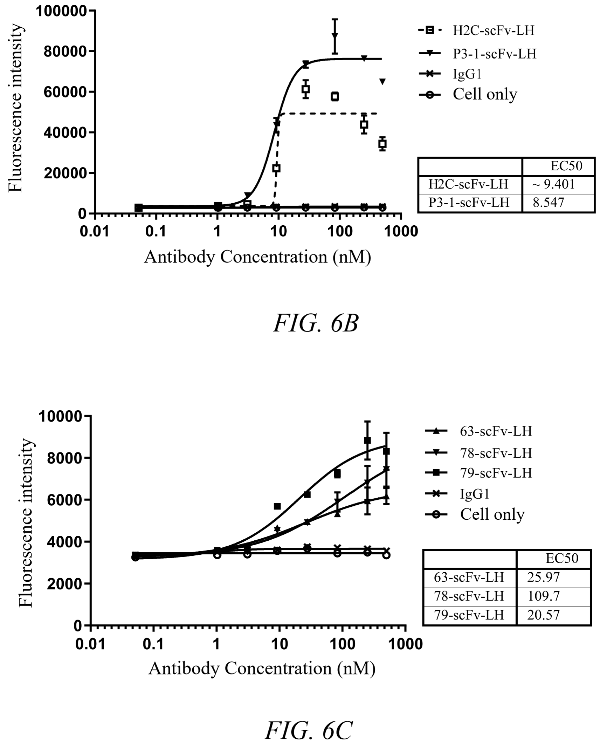

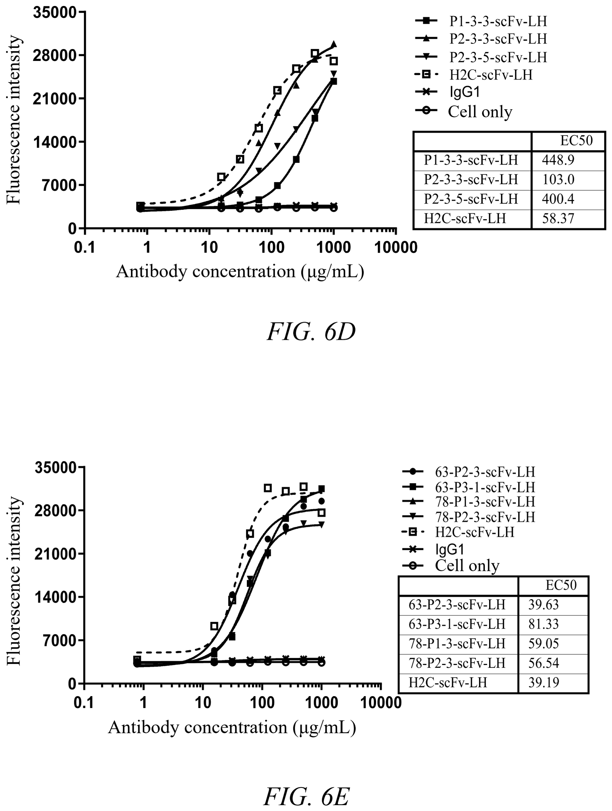

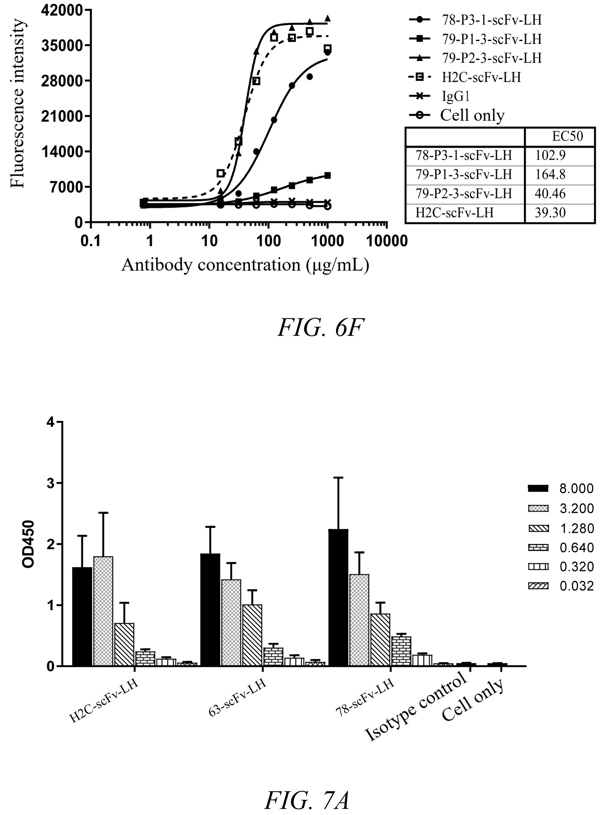

FIGs. 6A-6F show results of activating activity of antibodies on T cells based on the detection by a reporter gene assay. FIG. 6A shows the results of activating activity of P1-3-scFv-LH and P2-3-scFv-LH on T cells. FIG. 6B shows the results of activating activity of P3-1-scFv-LH on T cells. FIG. 6C shows the results of activating activity of 63-scFv-LH, 78-scFv-LH, and 79-scFv-LH on T cells. FIG. 6D shows the results of activating activity of P1-3-3-scFv-LH, P2-3-3-scFv-LH, and P2-3-5-scFv-LH on T cells. FIG. 6E shows the results of activating activity of 63-P2-3-scFv-LH, 63-P3-1-scFv-LH, 78-P1-3-scFv-LH, and 78-P2-3-scFv-LH on T cells. FIG. 6F shows the results of activating activity of 78-P3-1-scFv-LH, 79-P1-3-scFv-LH, and 79-P2-3-scFv-LH on T cells.

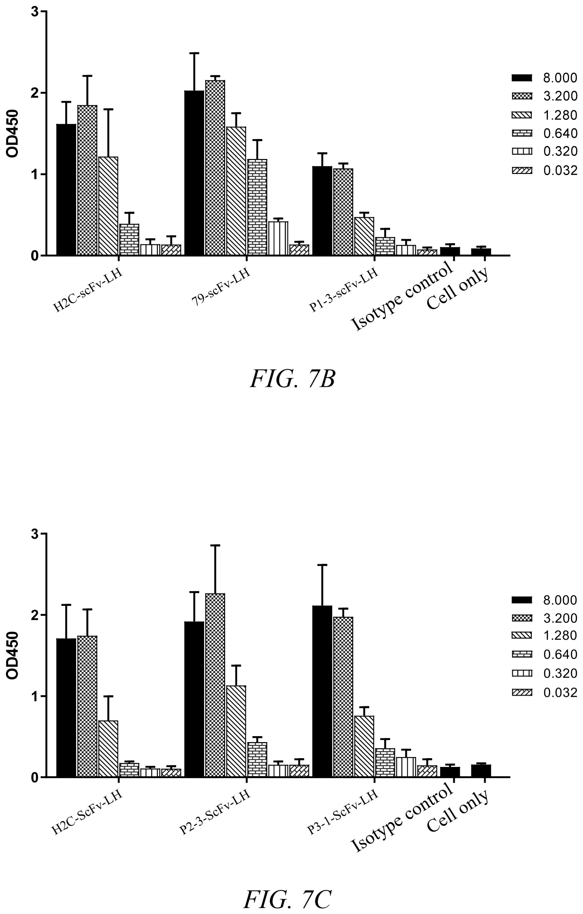

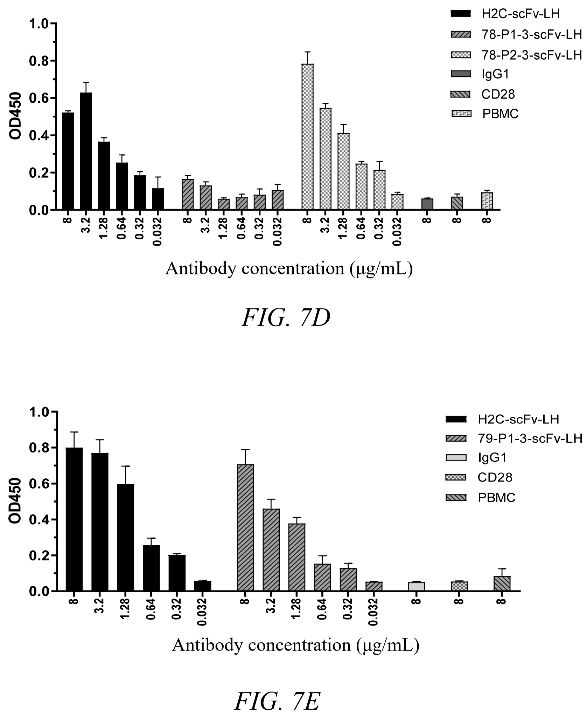

FIGs. 7A-7E show the ability of antibodies to induce cytokine IFN-γ secretion by ELISA. FIG. 7A shows the ability of 63-scFv-LH and 78-scFv-LH to induce cytokine IFN-γ secretion. FIG. 7B shows the ability of 79-scFv-LH and P1-3-scFv-LH to induce cytokine IFN-γ secretion. FIG. 7C shows the ability of P2-3-scFv-LH and P3-1-scFv-LH to induce cytokine IFN-γ secretion. FIG. 7D shows the ability of 78-P1-3-scFv-LH and 78-P2-3-scFv-LH to induce cytokine IFN-γ secretion. FIG. 7E shows the ability of 79-P1-3-scFv-LH to induce cytokine IFN-γ secretion.

### Detailed Description of Embodiments

Unless otherwise defined, all technical and scientific terms used herein have the same meanings as commonly understood by those of ordinary skill in the art to which the present invention pertains. All publications, patent applications, patents and other references mentioned herein are incorporated by reference in their entirety. In addition, the materials, methods and examples described herein are illustrative only and are not intended to be limiting. Other features, objectives and advantages of the present invention will be apparent from the description, the drawings and the appended claims.

### I. Definitions

For interpreting this description, the following definitions will apply and whenever appropriate, terms used in the singular will also include the plural and vice versa. It is to be understood that the terminology used herein is for the purpose of describing particular embodiments only, and is not intended to be limiting.

The term "about" when used in conjunction with a numerical value is meant to encompass numerical values within a range having a lower limit that is 5% smaller than the specified numerical value and an upper limit that is 5% larger than the specified numerical value.

As used herein, the term "and/or" means any one of optional items or two or more of the optional items.

Unless otherwise indicated, the term "contain" or "comprise" when used herein also encompasses situations consisting of the described elements, integers or steps. For example, when referring to an antibody variable region "containing" a particular sequence, it is intended to also encompass an antibody variable region consisting of that particular sequence.

As used herein, "T cell-activating antigen" refers to an antigenic determinant expressed on the surface of T lymphocytes, particularly cytotoxic T lymphocytes, which is capable of inducing T cell activation upon interaction with an antigen-binding molecule. Specifically, the interaction of the antigen-binding molecule with the T cell-activating antigen can induce T cell activation by triggering the signaling cascade of the T cell receptor complex. In a specific embodiment, the T cell-activating antigen is CD3.

As used herein, "T cell activation" refers to one or more cellular responses of T lymphocytes, particularly cytotoxic T lymphocytes, wherein the cellular responses are selected from: proliferation, differentiation, cytokine secretion, cytotoxic effector molecule release, cytotoxic activity and expression of activation markers. Assays for measuring T cell activation are known in the art.

As used herein, the terms "CD3 antibody", "antibody against CD3", "antibody specifically binding to CD3", "antibody specifically targeting CD3" and "antibody specifically recognizing CD3" are used interchangeably, and mean antibodies that can specifically bind to CD3.

The term "antibody" herein is used in the broadest sense, refers to a protein containing an antigen binding site, and encompasses natural antibodies and artificial antibodies of various structures, including but not limited to monoclonal antibodies, polyclonal antibodies, multispecific antibodies (e.g., bispecific antibodies), single chain antibodies, intact antibodies, and antibody fragments.

An "antibody fragment" or an "antigen-binding fragment" is used interchangeably herein to refer to a molecule different from an intact antibody, which comprises a portion of the intact antibody and binds to an antigen to which the intact antibody binds. Examples of antibody fragments include but are not limited to Fab, Fab', F(ab')₂, Fv, single chain Fv (scFv), single chain Fab or diabody.

The term "scFv" is an antibody fragment comprising a light chain variable region and a heavy chain variable region, wherein the light chain variable region and the heavy chain variable region are optionally linked in tandem by means of a flexible short polypeptide linker, and can be expressed as a single chain polypeptide. The scFv retains the specificity of the intact antibody from which it is derived. Unless otherwise indicated, as used herein, the scFv may have a VL variable region and a VH variable region in any order (e.g., relative to the N-terminus and C-terminus of a polypeptide), and the scFv may comprise a VL-linker-VH or may comprise a VH-linker-VL. An antibody that exhibits the same or similar binding affinity and/or specificity as a reference antibody refers to an antibody that has at least 50%, 60%, 70%, 80%, 90%, 95% or more of the binding affinity and/or specificity of the reference antibody. This can be determined by any method known in the art for determining binding affinity and/or specificity.

The term "variable region" or "variable domain" refers to the domain of the heavy or light chain of an antibody that is involved in binding of the antibody to an antigen. The variable domains of the heavy and light chains (VH and VL, respectively) of native antibodies generally have similar structures, with each domain containing four conserved framework regions (FRs) and three hypervariable regions (HVRs). HVRs typically comprise amino acid residues from hypervariable loops and/or complementarity determining regions (CDRs).

"Framework" or "FR" refers to variable domain residues other than hypervariable region (HVR) residues. The FR of the variable domain is typically composed of four FR domains: FR1, FR2, FR3 and FR4.

The "complementarity determining region" or "CDR region" or "CDR" is a region of an antibody variable domain which is hypervariable in the sequence and forms a structurally defined loop ("hypervariable loop") and/or contains an antigen contact residue ("antigen contact point"). CDR is primarily responsible for binding to an epitope of an antigen. CDRs of a heavy chain are generally referred to as CDR1, CDR2 and CDR3, numbered sequentially starting from N-terminus. In a given heavy chain variable region amino acid sequence, the precise amino acid sequence boundary of each CDR may be determined by using any one of many well-known antibody CDR assignment systems or a combination thereof, wherein the assignment system includes, for example: Chothia based on the three-dimensional structures of antibodies and the topology of CDR loops (Chothia et al., (1989) Nature 342: 877-883, Al-Lazikani et al., "Standard conformations for the canonical structures of immunoglobulins", Journal of Molecular Biology, 273, 927-948 (1997)), Kabat based on antibody sequence variability (Kabat et al., Sequences of Proteins of Immunological Interest, 4th edition, U.S. Department of Health and Human Services, National Institutes of Health (1987)), AbM (University of Bath), Contact (University College London), International ImMunoGeneTics database (IMGT) (http://imgt.cines.fr/), and the North CDR definition based on affinity propagation clustering using a large number of crystal structures.

Unless otherwise stated, in the present invention, the term "CDR" or "CDR sequence" encompasses CDR sequences determined by any one of the above methods.

CDR can also be determined on the basis of having the same AbM numbering position as a reference CDR sequence (such as any one of the exemplary CDRs of the present invention). In the present invention, when referring to antibody variable regions and specific CDR sequences (including heavy chain variable region residues), it is referred to as numbering positions according to the AbM numbering system.

Although CDRs vary from antibody to antibody, only a limited number of amino acid positions within the CDRs are directly involved in antigen binding. Using at least two of the Kabat, Chothia, AbM and Contact methods, a minimal overlap region can be determined, thereby providing a "minimal binding unit" for antigen binding. The minimal binding unit may be a sub-portion of a CDR. As will be apparent to a person skilled in the art, the residues of the remainder of the CDR sequence can be determined from the structure and protein folding of an antibody. Therefore, the present invention also contemplates variants of any of the CDRs given herein. For example, in a variant of a CDR, the amino acid residue of the minimal binding unit can remain unchanged, while the remaining CDR residues defined according to Kabat or Chothia or AbM can be replaced with conserved amino acid residues.

The "humanized" antibody refers to a chimeric antibody containing amino acid residues from non-human CDRs and amino acid residues from human FRs. In some embodiments, all or substantially all CDRs in a humanized antibody correspond to those in a non-human antibody, and all or substantially all FRs correspond to those in a human antibody. A humanized antibody may optionally contain at least a portion of an antibody constant region derived from a human antibody. A "humanized form" of an antibody (e.g., a non-human antibody) refers to an antibody that has undergone humanization.

The "human antibody" refers to an antibody having an amino acid sequence corresponding to that of an antibody produced by human or human cells or derived from a non-human source using human antibody repertoire or other human antibody coding sequences. This definition of the human antibody explicitly excludes a humanized antibody containing non-human antigen binding residues.

The term "Fc region" herein is used to define a C-terminal region of an immunoglobulin heavy chain, containing at least a portion of the constant region. The term includes natural sequence Fc regions and variant Fc regions. In certain embodiments, a human IgG heavy chain Fc region extends from Cys226 or Pro230 to the carbonyl terminus of the heavy chain. However, the C-terminal lysine (Lys447) of the Fc region may or may not be present. Unless otherwise stated, the amino acid residues in the Fc region or constant region are numbered according to the EU numbering system, also known as the EU index, as described in Kabat et al., Sequences of Proteins of Immunological Interest, 5th Ed. Public Health Service, National Institutes of Health, Bethesda, MD, 1991.

In certain embodiments, the Fc region of an immunoglobulin comprises two constant domains, namely CH2 and CH3, and in other embodiments, the Fc region of an immunoglobulin comprises three constant domains, namely CH2, CH3 and CH4.

The term "variable region" or "variable domain" refers to an antibody heavy or light chain domain that is involved in the binding of an antibody to an antigen. The heavy and light chain variable domains of a natural antibody generally have similar structures, with each domain containing four conserved framework regions (FRs) and three complementarity determining regions (CDRs) (see, for example, Kindt et al., Kuby Immunology, 6th ed., W.H.Freeman and Co., page 91 (2007)). A single VH or VL domain may be sufficient to confer an antigen binding specificity.

As used herein, the term "binding" or "specific binding" means that the binding is selective for an antigen and can be distinguished from unwanted or non-specific interactions. The ability of an antibody to bind to a particular antigen can be determined by enzyme-linked immunosorbent assay (ELISA), SPR or biolayer interferometry or other conventional binding assays known in the art.

As used herein, the term "monospecific" antibody refers to an antibody having one or more antigen binding sites, each of which binds to the same epitope of the same antigen.

As used herein, the term "multispecific" antibody refers to an antibody having at least two antigen binding sites, each of which binds to a different epitope of the same antigen or a different epitope of a different antigen. The antibodies provided herein are typically multispecific antibodies, such as bispecific antibodies. In one embodiment, provided herein is a bispecific antibody having binding specificities for CD3 and another antigen, such as a tumor antigen.

The term "flexible linking peptide" or "linking peptide" refers to a linking peptide composed of amino acids, such as glycine and/or serine residues, used alone or in combination, to link individual variable domains in an antibody. In one embodiment, the flexible linking peptide is a Gly/Ser linking peptide comprising an amino acid sequence (Gly₄Ser)n, wherein n is a positive integer equal to or greater than 1, for example, n is a positive integer from 1 to 7. In one embodiment the flexible linking peptide is (Gly₄Ser)₃ (SEQ ID NO: 70).

The "conjugate" refers to an antibody conjugated to one or more other substances (including but not limited to a cytotoxic agent or a label).

The terms "individual" and "subject" are used interchangeably, including mammals. Mammals include, but are not limited to, humans and non-human primates such as monkeys. Particularly, the individual or subject is a human.

The terms "tumor" and "cancer" are used interchangeably herein, and encompass solid tumors and liquid tumors.

The terms "cancer" and "cancerous" refer to the physiological illness in mammals in which cell growth is unregulated.

The term "tumor" refers to all neoplastic cell growth and proliferation, whether malignant or benign, and all pre-cancerous and cancerous cells and tissues. The terms "cancer", "cancerous" and "tumor" are not mutually exclusive when referred to herein.

The "isolated" antibody refers to having been separated from components of the natural environment thereof. In some embodiments, the anti-CD3 antibody is purified to more than 90% purity, as determined by, for example, electrophoresis (e.g., SDS-PAGE, isoelectric focusing (IEF) and capillary electrophoresis) or chromatography (e.g., ion exchange or reversed-phase HPLC). For a review of methods for evaluating the purity of an antibody, see, for example, Flatman et al., J. Chromatogr. B848: 79-87 (2007).

The "isolated" nucleic acid refers to a nucleic acid molecule that has been separated from components of the natural environment thereof. An isolated nucleic acid includes a nucleic acid molecule contained in cells that ordinarily contain the nucleic acid molecule, but the nucleic acid molecule is present extrachromosomally or at a chromosomal location that is different from its natural chromosomal location. The "isolated nucleic acid encoding anti-CD3 antibody" refers to one or more nucleic acid molecules encoding the chain or fragment of an anti-CD3 antibody, including such nucleic acid molecules in a single vector or separate vectors, and such nucleic acid molecules present at one or more locations in a host cell.

The calculation of sequence identity between sequences is performed as follows.

To determine the percent identity of two amino acid sequences or two nucleic acid sequences, the sequences are aligned for optimal comparison purposes (for example, gaps can be introduced in either or both of the first and second amino acid sequences or nucleic acid sequences for optimal alignment or non-homologous sequences can be discarded for comparison purposes). In a preferred embodiment, the length of a reference sequence aligned for comparison purposes is at least 30%, preferably at least 40%, more preferably at least 50%, 60% and even more preferably at least 70%, 80%, 90% and 100% of the length of the reference sequence. The amino acid residues or nucleotides at corresponding amino acid positions or nucleotide positions are then compared. When the position in the first sequence is occupied by the same amino acid residue or nucleotide as that at the corresponding position in the second sequence, the molecules are identical at that position.

The sequence comparison and the calculation of percent identity between two sequences can be achieved using a mathematical algorithm. In a preferred embodiment, the percent identity between two amino acid sequences is determined using the Needlema and Wunsch algorithm ((1970) J. Mol. Biol. 48: 444-453) in the GAP program that has been integrated into the GCG software package (available at http://www.gcg.com), and using the Blossum 62 matrix or the PAM250 matrix, with a gap weight of 16, 14, 12, 10, 8, 6 or 4 and a length weight of 1, 2, 3, 4, 5 or 6. In another preferred embodiment, the percent identity between two nucleotide sequences is determined using the GAP program in the GCG software package (available at http://www.gcg.com), and using the NWSgapdna.CMP matrix, with a gap weight of 40, 50, 60, 70 or 80 and a length weight of 1, 2, 3, 4, 5 or 6. A particularly preferred parameter set (and one parameter set that should be used unless otherwise stated) is the Blossum 62 scoring matrix with a gap penalty of 12, a gap extension penalty of 4 and a gap frameshift penalty of 5.

The percent identity between two amino acid sequences or nucleotide sequences can also be determined using the PAM120 weighted remainder table with a gap length penalty of 12 and a gap penalty of 4, and using the E. Meyers and W. Miller algorithm ((1989) CABIOS, 4:11-17) that has been incorporated into the ALIGN program (version 2.0).

Additionally or alternatively, the nucleic acid sequences and protein sequences described herein can further be used as "query sequences" to perform searches against public databases, for example, to identify other family member sequences or related sequences.

The terms "amino acid change" and "amino acid modification" are used interchangeably and refer to additions, deletions, substitutions, and other modifications of amino acids. Any combination of additions, deletions, substitutions, and other modifications of amino acids may be made, provided that the final polypeptide sequence has the desired properties.

The term "conservative sequence modification", "conservative sequence change" refers to an amino acid modification or change that does not significantly affect or alter the binding characteristics of an antibody or antibody fragment containing an amino acid sequence. Such conservative modifications include amino acid substitutions, additions, and deletions. Modifications can be introduced into the antibody or antibody fragment of the present invention by standard techniques known in the art, such as site-directed mutagenesis and PCR-mediated mutagenesis. A conservative substitution is an amino acid substitution in which an amino acid residue is replaced by an amino acid residue having a similar side chain. Families of amino acid residues having similar side chains have been defined in the art. These families include amino acids with basic side chains (e.g., lysine, arginine and histidine), acidic side chains (e.g., aspartic acid, and glutamic acid), uncharged polar side chains (e.g., glycine, asparagine, glutamine, serine, threonine, tyrosine, cysteine, and tryptophan), nonpolar side chains (e.g., alanine, valine, leucine, isoleucine, proline, phenylalanine, and methionine), β-side chains (e.g., threonine, valine, and isoleucine) and aromatic side chains (e.g., tyrosine, phenylalanine, tryptophan, and histidine).

The term "pharmaceutical composition" refers to a composition that is present in a form which allows the active ingredient contained therein to be biologically effective and does not contain additional ingredients that would be unacceptably toxic to a subject to which the pharmaceutical composition is administered.

The term "pharmaceutically acceptable carrier" refers to diluents, adjuvants (e.g. Freund's adjuvant (complete and incomplete)), excipients, buffers or stabilizers, and the like administered with an active substance.

The term "size-exclusion high-performance liquid chromatography" (i.e., SEC-HPLC) is a method used for antibody standards and quality control. This method mainly separates molecules based on their size or hydrodynamic radius differences. By SEC-HPLC, antibodies can be separated into three main forms: high molecular weight form (HMMS), main peak (mainly antibody monomer) and low molecular weight form (LMMS). Antibody purity can be calculated as the percentage of the main peak area to the sum of all peak areas on the chromatogram. Through the SEC-HPLC method, the percentage of antibody monomers in the preparation product can be measured, giving information on the content of soluble aggregates and shear products. For further description of the SEC-HPLC method, see, for example, J. Pharm. Scien., 83: 1645-1650, (1994); Pharm. Res., 11: 485 (1994); J. Pharm. Bio. Anal., 15: 1928 (1997); J. Pharm. Bio. Anal., 14: 1133-1140 (1986).

As used herein, the "treatment" refers to slowing, interrupting, blocking, relieving, stopping, reducing or reversing the progression or severity of an existing symptom, disorder, condition or disease. Desirable effects of treatment include, but are not limited to, preventing occurrence or recurrence of a disease, alleviating symptoms, diminishing any direct or indirect pathological consequences of a disease, preventing metastasis, decreasing the rate of disease progression, ameliorating or palliating disease state, and relieving or improving prognosis. In some embodiments, the antibody molecule of the present invention is used to delay the development of a disease or to slow the progression of a disease.

As used herein, the "prevention" includes the inhibition of the onset or development of a disease or disorder or symptoms of a particular disease or disorder. In some embodiments, a subject with a family history of cancer is a candidate for a prophylactic regimen. Generally, in the context of cancers, the term "prevention" refers to the administration of a drug prior to the onset of signs or symptoms of cancer, particularly before the onset of symptoms of cancer in a subject at risk of cancer.

The term "effective amount" refers to an amount or dose of the antibody or composition of the present invention which, upon administering to a patient in a single or multiple doses, produces the desired effect in the patient in need of treatment or prevention. The effective amount can be readily determined by the attending physician as a person skilled in the art with consideration of a variety of factors, such as the species of the mammal; body weight, age and general health condition; the specific disease involved; the extent or severity of the disease; the response of an individual patient; the specific antibody administered; the mode of administration; the bioavailability characteristics of the preparation administered; the selected dosing regimen; and use of any concomitant therapy.

The "therapeutically effective amount" refers to an amount effective to achieve the desired therapeutic result at a dose and for periods of time desired. The therapeutically effective amount of an antibody or an antibody fragment or a composition thereof can vary depending on a variety of factors such as disease state, age, sex and weight of an individual, and the ability of the antibody or antibody moiety to activate a desired response in an individual. The therapeutically effective amount is also an amount in which any toxic or harmful effect of an antibody or an antibody fragment or a composition thereof is less than a therapeutically beneficial effect. The "therapeutically effective amount" preferably inhibits measurable parameters (such as tumor growth rate and tumor volume) by at least about 20%, more preferably at least about 40%, even more preferably at least about 50%, 60% or 70%, and still more preferably at least about 80% or 90% relative to untreated subjects. The ability of an antibody or an antibody fragment or a composition thereof to inhibit a measurable parameter (such as cancer) can be evaluated in an animal model system for predicting the efficacy in a human tumor.

The "prophylactically effective amount" refers to an amount effective to achieve the desired prophylactic result at a dose and for periods of time desired. Generally, since a prophylactic dose is used in a subject before or during the earlier stage of a disease, the prophylactically effective amount will be less than the therapeutically effective amount.

The term "vector" as used herein when referring to a nucleic acid refers to a nucleic acid molecule capable of replicating another nucleic acid to which it is linked. The term includes the vector as a self-replicating nucleic acid structure and the vector incorporated into the genome of a host cell into which it has been introduced. Some vectors are capable of directing the expression of nucleic acids to which they are operably linked. Such vectors are referred to herein as "expression vectors".

The term "host cell" refers to a cell into which an exogenous polynucleotide is introduced, including the progeny of such cells. Host cells include "transformant" and "transformed cell", which include primary transformed cells and progeny derived therefrom regardless of the number of passages. The progeny may be not completely identical to a parent cell in terms of nucleic acid content, but may contain mutations. The mutant progeny that has the same function or biological activity screened or selected in the original transformed cell is included herein. Host cells are any type of cellular system that can be used to produce the antibody molecule of the present invention, including eukaryotic cells such as mammalian cells, insect cells and yeast cells; and prokaryotic cells, for example, *E. coli* cells. Host cells include cultured cells, and also include cells inside transgenic animals, transgenic plants or cultured plant tissues or animal tissues.

### II. The antibody specifically binding to CD3 molecule of the present invention

The present invention provides a group of cross-species anti-CD3 antibodies comprising a heavy chain variable region and a light chain variable region, wherein:
(a) the heavy chain variable region comprises, according to AbM numbering, HCDR1 as set forth in GFTFSKYAMN (SEQ ID NO: 51); HCDR2 as set forth in RIRSKYNNYATY (SEQ ID NO: 46); and HCDR3 as set forth in HGNFGNSYISYWAY (SEQ ID NO: 47); the light chain variable region comprises, according to AbM numbering, LCDR1 as set forth in GSSTGAVTSGYYPN (SEQ ID NO: 48); LCDR2 as set forth in GTKFLAP (SEQ ID NO: 49); and LCDR3 as set forth in ALWYSNRWV (SEQ ID NO: 50);
(b) the heavy chain variable region comprises, according to AbM numbering, HCDR1 as set forth in GFTFNKYAMN (SEQ ID NO: 45); HCDR2 as set forth in RIRSKYNNYATY (SEQ ID NO: 46); and HCDR3 as set forth in HGNFGNSYISYWAY (SEQ ID NO: 47); the light chain variable region comprises, according to AbM numbering, LCDR1 as set forth in GSSTGAVTSGYYPN (SEQ ID NO: 48); LCDR2 as set forth in GTKFLAP (SEQ ID NO: 49); and LCDR3 as set forth in ALWVDNRWV (SEQ ID NO: 55);
(c) the heavy chain variable region comprises, according to AbM numbering, HCDR1 as set forth in GFTFPKYAMN (SEQ ID NO: 52); HCDR2 as set forth in RIRSKYNNYETY (SEQ ID NO: 53); and HCDR3 as set forth in HGNFGNSYISYWAY (SEQ ID NO: 47); the light chain variable region comprises, according to AbM numbering, LCDR1 as set forth in GSSTGAVTSGYYPN (SEQ ID NO: 48); LCDR2 as set forth in GTHFLAP (SEQ ID NO: 54); and LCDR3 as set forth in ALWYSNRWV (SEQ ID NO: 50);
(d) the heavy chain variable region comprises, according to AbM numbering, HCDR1 as set forth in GFTFNKYAMN (SEQ ID NO: 45); HCDR2 as set forth in RIRSKYNNYATY (SEQ ID NO: 46); and HCDR3 as set forth in HGNFGNSYISYWAY (SEQ ID NO: 47); the light chain variable region comprises, according to AbM numbering, LCDR1 as set forth in GSSTGAVTSGYYPN (SEQ ID NO: 48); LCDR2 as set forth in GTKFLAP (SEQ ID NO: 49); and LCDR3 as set forth in ALWQENRWV (SEQ ID NO: 56);
(e) the heavy chain variable region comprises, according to AbM numbering, HCDR1 as set forth in GFTFSKYAMN (SEQ ID NO: 51); HCDR2 as set forth in RIRSKYNNYATY (SEQ ID NO: 46); and HCDR3 as set forth in HGNFGNSYISYWAY (SEQ ID NO: 47); the light chain variable region comprises, according to AbM numbering, LCDR1 as set forth in GSSTGAVTSGYYPN (SEQ ID NO: 48); LCDR2 as set forth in GTKFLAP (SEQ ID NO: 49); and LCDR3 as set forth in ALWVDNRWV (SEQ ID NO: 55);
(f) the heavy chain variable region comprises, according to AbM numbering, HCDR1 as set forth in GFTFSKYAMN (SEQ ID NO: 51); HCDR2 as set forth in RIRSKYNNYETY (SEQ ID NO: 53); and HCDR3 as set forth in HGNFGNSYISYWAY (SEQ ID NO: 47); the light chain variable region comprises, according to AbM numbering, LCDR1 as set forth in GSSTGAVTSGYYPN (SEQ ID NO: 48); LCDR2 as set forth in GTHFLAP (SEQ ID NO: 54); and LCDR3 as set forth in ALWYSNRWV (SEQ ID NO: 50); or
(g) the heavy chain variable region comprises, according to AbM numbering, HCDR1 as set forth in GFTFSKYAMN (SEQ ID NO: 51); HCDR2 as set forth in RIRSKYNNYATY (SEQ ID NO: 46); and HCDR3 as set forth in HGNFGNSYISYWAY (SEQ ID NO: 47); the light chain variable region comprises, according to AbM numbering, LCDR1 as set forth in GSSTGAVTSGYYPN (SEQ ID NO: 48); LCDR2 as set forth in GTKFLAP (SEQ ID NO: 49); and LCDR3 as set forth in ALWQENRWV (SEQ ID NO: 56).

In some embodiments, the antibody that binds to a CD3 molecule of the present invention bind to CD3 of human and non-human primates, and the non-human primates include New World monkeys (Marmoset, Callithrix jacchus; Saguinus oedipus; and Saimiri sciureus) and Old World monkeys (Macaca fascicularis, also known as Cynomolgus Monkey; or Macaca mulatta, also known as Rhesus Monkey). Sequence analysis confirmed that humans and non-human primates have highly homologous sequence segments at the N-terminus of the CD3ε extracellular domain.

In some embodiments, the antibody that binds to CD3 molecule of the present invention comprises a heavy chain variable region and a light chain variable region that specifically bind to CD3, wherein:
(a) a heavy chain variable region as set forth in SEQ ID NO: 26 and a light chain variable region as set forth in SEQ ID NO: 27;
(b) a heavy chain variable region as set forth in SEQ ID NO: 28 and a light chain variable region as set forth in SEQ ID NO: 29;
(c) a heavy chain variable region as set forth in SEQ ID NO: 30 and a light chain variable region as set forth in SEQ ID NO: 2;
(d) a heavy chain variable region as set forth in SEQ ID NO: 1 and a light chain variable region as set forth in SEQ ID NO: 31;
(e) a heavy chain variable region as set forth in SEQ ID NO: 32 and a light chain variable region as set forth in SEQ ID NO: 33;
(f) a heavy chain variable region as set forth in SEQ ID NO: 1 and a light chain variable region as set forth in SEQ ID NO: 34;
(g) a heavy chain variable region as set forth in SEQ ID NO: 30 and a light chain variable region as set forth in SEQ ID NO: 27;
(h) a heavy chain variable region as set forth in SEQ ID NO: 35 and a light chain variable region as set forth in SEQ ID NO: 29;
(i) a heavy chain variable region as set forth in SEQ ID NO: 30 and a light chain variable region as set forth in SEQ ID NO: 29;
(j) a heavy chain variable region as set forth in SEQ ID NO: 28 and a light chain variable region as set forth in SEQ ID NO: 36;
(k) a heavy chain variable region as set forth in SEQ ID NO: 30 and a light chain variable region as set forth in SEQ ID NO: 31;
(l) a heavy chain variable region as set forth in SEQ ID NO: 37 and a light chain variable region as set forth in SEQ ID NO: 38;
(m) a heavy chain variable region as set forth in SEQ ID NO: 39 and a light chain variable region as set forth in SEQ ID NO: 40;
(n) a heavy chain variable region as set forth in SEQ ID NO: 41 and a light chain variable region as set forth in SEQ ID NO: 33;
(o) a heavy chain variable region as set forth in SEQ ID NO: 26 and a light chain variable region as set forth in SEQ ID NO: 42;
(p) a heavy chain variable region as set forth in SEQ ID NO: 28 and a light chain variable region as set forth in SEQ ID NO: 43; or
(q) a heavy chain variable region as set forth in SEQ ID NO: 30 and a light chain variable region as set forth in SEQ ID NO: 44.

In some embodiments, the antibody that binds to CD3 molecule of the present invention is an IgG1, IgG2, IgG3, or IgG4 antibody; preferably an IgG1 or IgG4 antibody; more preferably, an IgG1 antibody, for example, a human IgG1 antibody.

In some embodiments, the antibody that binds to a CD3 molecule of the present invention has one or more of the following properties:
(i) a monomer purity greater than 90%, preferably greater than 92%, 94%, 96%, 97%, as measured by SEC-HPLC;
(ii) a purity greater than 90%, preferably greater than 91%, 92%, or 93%, as measured by reducing or non-reducing SDS-PAGE;
(iii) specifically binding to CD3 expressed on a cell surface.

In some embodiments, the present invention provides a nucleic acid encoding any of the above antibody that binds to a CD3 molecule or the fragment thereof or any one of the chains thereof. In one embodiment, provided is a vector containing the nucleic acid. In one embodiment, the vector is an expression vector. In one embodiment, provided is a host cell containing the nucleic acid or the vector. In one embodiment, the host cell is a eukaryotic cell. In another embodiment, the host cell is selected from a yeast cell, a mammalian cell (such as a CHO cell or a 293 cell), or other cells suitable for the preparation of antibodies or antigen-binding fragments thereof. In another embodiment, the host cell is a prokaryotic cell.

For example, the nucleic acid of the present invention comprises a nucleic acid encoding the antibody that binds to a CD3 molecule of the present invention. In some embodiments, provided are one or more vectors containing the nucleic acid. In one embodiment, the vector is an expression vector, for example, a eukaryotic expression vector. Vectors include, but are not limited to, viruses, plasmids, cosmids, λ phages or yeast artificial chromosomes (YAC). In one embodiment, the vector is a pcDNA3.4 expression vector.

Once an expression vector or a DNA sequence has been prepared for expression, the expression vector can be transfected or introduced into a suitable host cell. Various techniques, such as protoplast fusion, calcium phosphate precipitation, electroporation, retroviral transduction, viral transfection, gene gun, lipid-based transfection or other conventional techniques, can be used to achieve this purpose. In the case of protoplast fusion, cells are grown in a culture medium and screened for appropriate activity. Methods and conditions for culturing the resulting transfected cells and for recovering the resulting antibody molecules are known to those skilled in the art and can be varied or optimized on the basis of the present description and methods known in the prior art, depending on the particular expression vector and mammalian host cell used.

Additionally, cells that have stably incorporated DNA into their chromosomes can be selected by introducing one or more markers that allow selection of transfected host cells. Markers may, for example, provide prototrophy, biocidal resistance (such as antibiotics) or resistance to heavy metals (such as copper) to auxotrophic hosts. A selectable marker gene can be directly linked to a DNA sequence to be expressed or introduced into the same cell by co-transformation. Additional elements may also be required for optimal synthesis of mRNA. These elements may include splicing signals, as well as transcription promoters, enhancers and termination signals.

In one embodiment, provided is a host cell containing the polynucleotide of the present invention. In some embodiments, provided is a host cell containing the expression vector of the present invention. In some embodiments, the host cell is selected from a yeast cell, a mammalian cell or other cells suitable for the preparation of antibodies. Suitable host cells include prokaryotic microorganisms, such as *E. coli.* Host cells can also be eukaryotic microorganisms such as filamentous fungi or yeasts, or various eukaryotic cells such as insect cells. Vertebrate cells can also be used as hosts. For example, mammalian cell lines engineered to be adapted to grow in suspension can be used. Examples of useful mammalian host cell lines include monkey kidney CV1 line transformed by SV40 (COS-7); human embryonic kidney lines (HEK293 or 293F cells), 293 cells, baby hamster kidney cells (BHK), monkey kidney cells (CV1), African green monkey kidney cells (VERO-76), human cervical cancer cells (HELA), canine kidney cells (MDCK), Buffalo rat liver cells (BRL 3A), human lung cells (W138), human liver cells (HepG2), Chinese hamster ovary cells (CHO cells), CHO-S cells, NSO cells, and myeloma cell lines such as Y0, NS0, P3X63 and Sp2/0. For a review of mammalian host cell lines suitable for protein production, see, for example, Yazaki and Wu, Methods in Molecular Biology, Vol. 248 (ed. B.K.C. Lo, Humana Press, Totowa, NJ), pp. 255-268 (2003). In a preferred embodiment, the host cell is a CHO cell or an HEK293 cell.

In one embodiment, the present invention provides a method for preparing an antibody that binds to CD3 molecule, wherein the method comprises culturing a host cell containing a nucleic acid encoding the antibody that binds to CD3 molecule or an expression vector containing the nucleic acid under conditions suitable for expressing the nucleic acid encoding the antibody that binds to CD3 molecule, and optionally isolating the antibody that binds to CD3 molecule. In a certain embodiment, the method further comprises recovering the antibody that binds to CD3 molecule from the host cell (or a host cell culture medium).

The antibody that binds to CD3 molecule of the present invention prepared as described herein can be purified by techniques known in the prior art, such as high-performance liquid chromatography, ion exchange chromatography, gel electrophoresis, affinity chromatography and size exclusion chromatography. The actual conditions used to purify a particular protein also depend on factors such as net charge, hydrophobicity and hydrophilicity, which will be apparent to a person skilled in the art. The purity of the antibody that binds to CD3 molecule of the present invention can be determined by any of a variety of well-known analytical methods, including steric exclusion chromatography, gel electrophoresis, high performance liquid chromatography, etc.

The antibody that binds to CD3 molecule provided herein can be identified, screened or characterized for its physical/chemical properties and/or biological activities by a variety of assays known in the art. In one aspect, the antibody that binds to CD3 molecule of the present invention is tested for its binding activity to an antigen, for example, by known methods such as FACS, ELISA or Western blotting. The binding to CD3 can be determined using methods known in the art, and exemplary methods are disclosed herein. In some embodiments, the binding of the antibody that binds to CD3 molecule of the present invention to cell surface CD3 (e.g., human CD3) is determined using FACS.

The present invention also provides an assay for identifying the antibody that binds to CD3 molecule having biological activity. The biological activity may include, for example, activation of the NF-κB signaling pathway, cytokine IFN-γ secretion, and the like.

Cells for use in any of the above *in-vitro* assays include cell lines that naturally express CD3 or are engineered to express CD3. The cell line engineered to express CD3 is a cell line that normally does not express CD3, but expresses CD3 upon transfection of DNA encoding CD3 into the cell.

### III. Fusions, conjugates, and bispecific antibodies

The present invention provides a fusion, conjugate and bispecific antibody comprising the antibody of the present invention. The fusion or conjugate can be produced by fusing or conjugating the antibody of the present invention to a heterologous molecule. The bispecific antibody can be produced by fusing the antibody of the present invention to another antibody capable of binding to a target cell antigen.

In some embodiments, the antibody polypeptide of the present invention may be fused or conjugated to one or more heterologous molecules, wherein the heterologous molecules include, but are not limited to, proteins/polypeptides/peptides, markers, drugs, and cytotoxic agents. Methods for fusing or conjugating proteins, polypeptides or peptides or chemical molecules to antibodies are known in the art. See, for example, U.S. Pat. Nos. 5,336,603 and 5,622,929, and EP 367,166.

In one embodiment, the antibody of the present invention is recombinantly fused to a heterologous protein or polypeptide or peptide to form a fusion protein. In yet another embodiment, the antibody of the present invention is conjugated to a protein molecule or a non-protein molecule to produce a conjugate.

In some embodiments, the antibody of the present invention in the form of a full-length antibody or antibody fragment may be fused or conjugated to a heterologous molecule.

The linker can be used to covalently link different entities in the fusion and/or conjugate of the present invention. The linker includes a chemical linker or a single-chain peptide linker. In some embodiments, the antibody of the present invention is fused to other peptides or proteins via a peptide linker. In some embodiments, the antibody of the present invention is conjugated to other molecules, such as markers or drug molecules, via a chemical linker.

The peptide linker of the present invention includes a peptide composed of amino acid residues. Such a linker peptide is typically flexible, allowing the antigen-binding moiety to which it is linked to move independently. The length of the linker peptide can be easily determined by those skilled in the art according to actual circumstances, for example, at least 4-15 amino acids long, or longer, for example, about 20-25 amino acids long.

In some embodiments, the anti-CD3 antibody of the present invention is fused to another antibody capable of binding to a target cell antigen to produce a bispecific antibody. In some embodiments, the antibody that binds to a target cell antigen is directed against an antigen associated with a pathological condition, such as an antigen present on a tumor cell or a virus-infected cell. Suitable antigens are cell surface antigens such as, but not limited to, cell surface receptors. In a specific embodiment, the antigen is a human antigen. In a specific embodiment, the target cell antigen is selected from, for example, folate receptor 1 (FolR1), mucin-1 (MUC1), B cell maturation antigen (BCMA), CD19, CD33, EGFR, EpCAM, HER2, CEA, EphA2, and ROR1.

### IV. Methods and compositions for treatment

The present invention provides a method of treating a disease, comprising administering to a subject an effective amount of the anti-CD3 antibody or antigen-binding fragment thereof of the present invention, or the fusion, conjugate or bispecific antibody of the present invention.

The anti-CD3 antibody or antigen-binding fragment thereof of the present invention may be administered to a cell in culture in vitro or ex vivo, or to a subject in vivo, to treat or prevent a condition such as cancer, an inflammatory disease or an autoimmune disease.

In some embodiments, the present invention provides a methods for treating or preventing cancer using the anti-CD3 antibody or antigen-binding fragment thereof of the present invention, wherein appropriate activation of T cells by the anti-CD3 antibody or antigen-binding fragment thereof of the present invention results in treatment or prevention, and the method comprises administering to a subject in need thereof a therapeutically effective amount or prophylactically effective amount of the anti-CD3 antibody or antigen-binding fragment thereof or the pharmaceutical composition of the present invention.

In some embodiments, the present invention provides a methods for treating or preventing cancer using a bispecific antibody that specifically binds to CD3 and a cancer-associated antigen, the anti-CD3 antibody or antigen-binding fragment thereof of the present invention contained in the bispecific antibody specifically binds to CD3, the other part of the bispecific antibody specifically binds to the cancer-associated antigen, such as folate receptor 1 (FolR1), mucin-1 (MUC1), B cell maturation antigen (BCMA), CD19, CD33, EGFR, EpCAM, HER2, CEA, EphA2, and ROR1. In some embodiments, the cancer treated or prevented by the bispecific antibody is, for example, breast cancer, prostate cancer, non-small cell lung cancer, bladder cancer, ovarian cancer, gastric cancer, colorectal cancer, cervical cancer, pancreatic cancer, testicular cancer, melanoma, soft tissue cancer (e.g. synovial sarcoma), esophageal cancer, head and neck squamous cell carcinoma, B-cell lymphoma, chronic lymphocytic leukemia, or acute lymphocytic leukemia.

It will be appreciated that the CD3 antibody, the fusion, the conjugate, and the bispecific antibody of the present invention can be administered in combination with other therapeutic modalities for the treatment of the above-described diseases. Such other therapeutic modalities include therapeutic agents, radiotherapy, chemotherapy, transplantation, immunotherapy, and the like. In some embodiments, the CD3 antibody, the fusion, the conjugate, and the bispecific antibody of the present invention are used in combination with other therapeutic agents. Exemplary therapeutic agents include chemotherapeutic agents, radiotherapeutic agents, other therapeutic antibodies, or other active agents and adjuvant agents, such as anti-tumor drugs.

The following examples are described to assist in the understanding of the present invention. The examples are not intended and should not be construed to limit the scope of protection of the present invention in any way.

### Examples

### Example 1: Preparation and identification of parent antibody protein and antigen protein

### 1.1 Preparation of parent antibody protein

Preparation of control antibody: In the present application, an anti-CD3E AA1-27 antibody H2C was used as a positive control antibody. Gene synthesis of a fragment of interest was performed by Gene Universal (Anhui) Co., Ltd. according to sequences disclosed in Patent Publication No. WO 2008119567 A2 (amino acid sequences of a heavy chain variable region and a light chain variable region of an H2C monoclonal antibody were as set forth in SEQ ID NO: 1 and SEQ ID NO: 2, respectively). Nucleotide sequences of the heavy chain variable region and the light chain variable region of the H2C antibody were synthesized. Then, a eukaryotic expression vector pcDNA3.4-TOPO (Invitrogen) was constructed by a homologous recombination method. The constructed recombinant protein expression vectors were respectively transformed into E. coli DH5α cultured at 37°C overnight, and then an endotoxin-free plasmid extraction kit (OMEGA, D6950-01) was used to extract plasmids, so as to obtain a desired expression vector expressing the H2C monoclonal antibody. By using an ExpiFectamine^{™} CHO transfection kit (Thermo Fisher, A29129), the expression vector was transfected into HEK293 cells (ATCC^{®} CRL-1573^{™}). 7 days after transfection, a cell culture supernatant was collected and centrifuged at 15000 g for 10 min. After the obtained supernatant was filtered through a 0.22 µm filter membrane, the antibody in the supernatant was subjected to affinity purification with a Protein A/G affinity chromatography column. 100 mM glycinate (pH 3.0) was used to elute the antibody of interest, and the eluted antibody was buffer-exchanged into a PBS buffer through an ultrafiltration concentration tube (Millipore, UFC901096).

### 1.2 Preparation and identification of antigen protein

Preparation of antigen protein: A human IgG1 Fc (SEQ ID NO: 4) tag was added to the C-terminus of the sequence of human CD3E protein CD3E AA1-27 (see WO 2008119565 A2, SEQ ID NO: 3) by genetic manipulation at a gene level. The obtained nucleic acid sequence was constructed into a pcDNA3.4-TOPO vector, the vector was then transformed into E. coli DH5α, which was cultured at 37°C overnight, and then the endotoxin-free plasmid extraction kit (OMEGA, D6950-01) was used to extract the plasmids. The obtained plasmids were transiently transfected into HEK293 cells (ATCC^{®} CRL-1573^{™}) with an ExpiFectamine^{™} 293 transfection kit (Gibco^{™}, A14524). After 7 days of expression, the cell culture supernatant was collected to perform affinity purification by using the Protein A/G affinity chromatography column. After purification, the protein of interest was eluted using 100 mM glycinate (pH = 3.0), concentrated and subjected to buffer exchange, and finally an antigen protein (huCD3E-AA1-27-huFc) was obtained.

Identification of antigen: The prepared antigen (huCD3E-AA1-27-huFc) was detected using the antibody H2C (IgG1) obtained in example 1.1 above and qualified by quality inspection. The specific method was as follows: An ELISA plate was coated with 2 µg/mL huCD3E-AA1-27-huFc at 4°C overnight; the plate was washed 3 times, and then was blocked with 5% skim milk formulated in PBS for 1 h at room temperature; After the plate was washed 3 times, antibody H2C serially diluted with PBS was added and incubated at room temperature for 1 h; After washing the plate, a secondary antibody Anti-human-IgG-Kappa+Lambda-HRP (Millipore, AP502P+AP506P) diluted with PBS (1 : 6000) was added and incubated at room temperature for 1 h. After washing the plate 6 times, TMB was added to develop color for 5-20 min. The color development reaction was terminated, OD450 data was read with a microplate reader, and the data was processed and plotted with Graphpad prism. The results are shown in FIG. 1. Antibody H2C (IgG1) binds well to the antigen huCD3E-AA1-27-huFc constructed and expressed in example 1.2, with an antigen-binding activity of EC₅₀ of 0.01 µg/mL.

### 1.3 Construction of huCD3D-CD3E-CHO-K1 overexpression cell line

The nucleotide sequences encoding human CD3D (Uniprot ID: P04234, SEQ ID NO: 5) and CD3E (Uniprot ID: P07766, SEQ ID NO: 6) were respectively constructed into a pLVX-puro plasmid (Clontech, Cat#632164). The obtained plasmid was electroporated into CHO-K1 cells (ATCC) using an electroporator (Invitrogen, Neon^{™} Transfection System, MP922947). After electroporation, the obtained cells were transferred to antibiotics-free F-12K media (Gibco) containing 10% FBS by volume (Gibco, 15140-141). Subsequently, the cells were seeded into a 10 × 10 cm cell culture dish and cultured for 48 h, and then the cells were plated at a density of 0.5E+4 cells/well on average to a 96-well cell culture plate. Puromycin (Gibco, A111138-03) was added at a final concentration of 8 µg/mL as the screening pressure. After around 2 weeks, the growth of cell line clones was observed, and the cloned cell lines were picked and identified.

Flow cytometric identification of huCD3D-CD3E-CHO-K1 cells: the foregoing cell strains in a logarithmic growth phase were digested and plated into a 96-well plate, and washed with a FACS buffer (1 × PBS buffer containing 2% FBS by volume), and then the primary antibody (H2C) serially diluted with PBS was added and incubated at 4°C for 30 min; after the plate was washed, the formulated fluorescent secondary antibody Anti human IgG Fc (abcam, 98596) was added and incubated at 4°C for 30 min; finally, detection was performed by a flow cytometer (Beckman, CytoFLEXAOO-1-1102). The detection results showed that huCD3D-CD3E-CHO-K1 cell lines with high expression of human CD3D&CD3E on the cell surface were obtained.

### 1.4 Construction of NF-κB-Jurkat overexpression cell line

The pGL4.30 plasmid (Promega, #E8481) containing the NF-κB-re nucleic acid sequence was electroporated into Jurkat cells (ATCC^{®} TIB-152) to obtain the NF-κB-Jurkat cell line. The electroporated cells were transferred to 1640 medium (Thermofisher, 11875093) and cultured in a 37°C cell incubator for 48 h. Then, 1000 cells/well were plated into a 96-well plate, and puromycin was added at a final concentration of 2 µg/mL for pressure screening. Single cell clones grown on the 96-well plate were picked and subjected to expansion culture, and then the catalytic substrate of luciferase was added for fluorescence signal detection and identification to obtain the NF-κB-Jurkat cell line.

### 1.5 Construction of huCD3E-AA1-27-HEK293 overexpression cell line

The nucleotide sequence encoding human CD3E AA1-27 (SEQ ID NO: 3) was constructed into the pLVX-puro plasmid (Clontech, Cat #632164). Then, the obtained plasmid was electroporated into HEK293 cells (ATCC^{®} CRL-1573^{™}) using an electroporator (Invitrogen, NeonTM Transfection System, MP922947). After electroporation, the obtained cells were respectively transferred to an antibiotics-free DMEM culture medium (Gibco, 11995065) containing 10% FBS by volume (Gibco, 15140-141). Then, the cells were transferred into a 10 × 10 cm cell culture dish and cultured for 48 h, and then the cells were seeded into a 96-well cell culture plate at an average density of 0.51E+4 cells/well. Puromycin at a final concentration of 2 µg/mL was added as a screening pressure, and the cloned cell strains were picked for identification in about 2 weeks.

Flow cytometric identification of huCD3E-AA1-27-HEK293 overexpression cells: Cells of the foregoing cell strains in a logarithmic growth phase were digested and plated into a 96-well plate, and washed with a FACS buffer (1 × PBS buffer containing 2% FBS by volume), and then the primary antibody (H2C) obtained in example 1.1 serially diluted with a PBS was added and incubated at 4°C for 30 min; after the plate was washed, the formulated fluorescent secondary antibody Anti human IgG Fc (abcam, 98596) was added and incubated at 4°C for 30 min; finally, detection was performed by a flow cytometer (Beckman, CytoFLEXAOO-1-1102). The detection results showed that a huCD3E-AA1-27-HEK293 cell line with high expression of human CD3E AA1-27 on the cell surface was obtained.

### 1.6 Construction of CynoCD3E-HEK293 overexpression cell line

The nucleotide sequence encoding monkey CD3E (Uniprot ID: Q95LI5, SEQ ID NO: 81) was constructed into the pLVX-puro plasmid (Clontech, Cat #632164). Then, the obtained plasmid was electroporated into HEK293 cells (ATCC^{®} CRL-1573^{™}) using an electroporator (Invitrogen, NeonTM Transfection System, MP922947). After electroporation, the obtained cells were respectively transferred to an antibiotics-free DMEM culture medium (Gibco, 11995065) containing 10% FBS by volume (Gibco, 15140-141). Then, the cells were transferred into a 10 × 10 cm cell culture dish and cultured for 48 h, and then the cells were seeded into a 96-well cell culture plate at an average density of 0.5E+4 cells/well. Puromycin at a final concentration of 2 µg/mL was added as a screening pressure, and the cloned cell strains were picked for identification in about 2 weeks.

Flow cytometric identification of CynoCD3E-HEK293 overexpression cells: Cells of the foregoing cell strains in a logarithmic growth phase were digested and plated into a 96-well plate, and washed with a FACS buffer (1 × PBS buffer containing 2% FBS by volume), and then the primary antibody (H2C) obtained in example 1.1 serially diluted with a PBS was added and incubated at 4°C for 30 min; after the plate was washed, the formulated fluorescent secondary antibody Anti human IgG Fc (abcam, 98596) was added and incubated at 4°C for 30 min; finally, detection was performed by a flow cytometer (Beckman, CytoFLEXAOO-1-1102). The detection results showed that a CynoCD3E-HEK293 cell line with high expression of monkey CD3E on the cell surface was obtained.

### Example 2 Antibody engineering of anti-CD3 monoclonal antibody

### 2.1 Humanization

In this example, scFv of the antibody H2C (H2C-scFv, SEQ ID NO: 7, obtained by ligating VL and VH of H2C with a linker peptide (SEQ ID NO: 70)) was subjected to humanization, so as to obtain different affinity candidate antibodies with higher degree of humanization. Humanization was based on M13 phage display technology, and degenerate primers were used to construct a site mutation library of back mutation.

The specific method for constructing the libraries was as follows: first, primers containing point mutations were synthesized (GENEWIZ Suzhou); and second, the antibody to be engineered (also called parent antibody) H2C-scFv was used as a PCR amplification template to amplify the sequences of FR regions comprising designed mutations, respectively; fragments comprising different FR region mutations were ligated by bridge PCR, the point-mutated antibody was then ligated into a phage display vector by double enzyme digestion (Hind III and Not I) and double-sticky-end ligation, and finally the antibody sequence with mutation sites was transformed into *E. coli* SS320 by electroporation. The transformed E. coli SS320 bacterial solution was diluted at a concentration gradient, and coated on a 2-YT solid plate with ampicillin resistance to obtain single colony, and a bacterial solution of the single colony was prepared.

After ELISA screening and sequencing analysis of the monoclonal bacterial liquid with the antigen protein huCD3E AA1-27-huFc, the engineered molecules P1-3-scFv (SEQ ID NO: 8), P2-3-scFv (SEQ ID NO: 9), P3-1-scFv (SEQ ID NO: 10), P1-1-scFv (SEQ ID NO: 71), P2-1-scFv (SEQ ID NO: 72), P2-2-scFv (SEQ ID NO: 73) and P3-4-scFv (SEQ ID NO: 74) were obtained.

### 2.2 Affinity maturation

In this example, the antibody H2C-scFv was subjected to affinity maturation to obtain candidate antibodies of different affinities. The affinity maturation was based on the M13 phage display technology, CDR region mutations were introduced using codon-based primers (in the primer synthesis process, a single codon was composed of NNK), and 4 phage display libraries were constructed. Library 1 and library 2 were single-point combinatorial mutations, wherein library 1 was LCDR1+LCDR3+HCDR3 combinatorial mutation, and library 2 was LCDR2+HCDR1+HCDR2 combinatorial mutation; and library 3 and library 4 were double-point saturation mutations, wherein library 3 was double-point saturation mutation of LCDR3, and library 4 was double-point saturation mutation of HCDR3.

The specific method for constructing the libraries was as follows: first, primers containing point mutations were synthesized (GENEWIZ Suzhou); and second, the scFv of the antibody to be engineered (also called parent antibody) H2C was used as a PCR amplification template to amplify the sequences of CDR regions comprising designed mutations, respectively; fragments comprising different CDR mutations were assembled by bridge PCR, the point-mutated antibody was then ligated into a phage display vector by double enzyme digestion (Hind III and Not I) and double-sticky-end ligation, and finally the antibody sequence with mutation sites was transformed into *E. coli* SS320 by electroporation.

The operation processes of library capacity calculation, phage library preparation and library screening were detailed in example 2.1. The transformed E. coli SS320 bacterial solution was diluted at a concentration gradient, and coated on a 2-YT solid plate with ampicillin resistance to obtain single colony, and a bacterial solution of the single colony was prepared. After ELISA screening and sequencing analysis using the antigen protein huCD3E AA1-27-huFc, the engineered molecules 63-scFv (SEQ ID NO: 11), 78-scFv (SEQ ID NO: 12), 79-scFv (SEQ ID NO: 13), 16-scFv (SEQ ID NO: 75), 23-scFv (SEQ ID NO: 76), 34-scFv (SEQ ID NO: 77), 43-scFv (SEQ ID NO: 78), 67-scFv (SEQ ID NO: 79) and 73-scFv (SEQ ID NO: 80) were obtained.

### Example 3 Construction, expression and purification of candidate antibody

### 3.1 Construction of plasmids

The C-terminus of the scFv sequence of the engineered molecule obtained by screening was linked to the coding sequence of the constant region Fc of human IgG1 (SEQ ID NO: 25), respectively, to construct the coding sequence of the obtained antibody. These scFv antibody sequences were inserted into eukaryotic expression vector plasmid pcDNA3.4-TOPO (Invitrogen), which was transformed into E. coli DH5α, which was cultured at 37°C overnight. An endotoxin-free plasmid extraction kit (OMEGA, D6950-01) was used for plasmid extraction to obtain an endotoxin-free antibody plasmid for eukaryotic expression. The antibody molecules linked to the constant region Fc of human IgG1 were named P1-3-scFv-LH, P2-3-scFv-LH, P3-1-scFv-LH, P1-1-scFv-LH, P2-1-scFv-LH, P2-2-scFv-LH, P3-4-scFv-LH, 63-scFv-LH, 78-scFv-LH, 79-scFv-LH, 16-scFv-LH, 23-scFv-LH, 34-scFv-LH, 43-scFv-LH, 67-scFv-LH and 73-scFv-LH, respectively, where "LH" refers to the arrangement of the variable domains in the order VL-VH.

### 3.2 Expression and purification of candidate antibody

The candidate antibody was expressed using ExpiCHO transient expression system (Thermo Fisher, A29133), and the specific method was as follows. On the day of transfection, a cell density of around 7 × 10⁶ to 1 × 10⁷ viable cells/mL and a cell viability of > 98% were confirmed, at which time, the cells were adjusted to a final concentration of 6 × 10⁶ cells/mL using fresh ExpiCHO expression medium pre-warmed at 37°C. The plasmid constructed in example 3.1 was diluted using OptiPROTM SFM pre-chilled at 4°C (1 µg of the plasmid was added to 1 mL of the medium), meanwhile, ExpiFectamineTMCHO was diluted using OptiPROTMSFM, the two dilutions were mixed in equal volumes and mixed uniformly by gently pipetting to prepare an ExpiFectamineTMCHO/plasmid DNA mixed liquid, the mixed liquid was incubated at room temperature for 1-5 min, and slowly added to the prepared cell suspension while gentle shaking, and finally the cells were placed in a cell culture shaker and cultured at 37°C and 8% CO₂.

18-22 h after transfection, ExpiCHOTMEnhancer and ExpiCHOTMFeed were added to the culture liquid, and the shake flask was placed in a 32°C shaker under 5% CO₂ for further culture. On day 5 after transfection, ExpiCHOTMFeed with the same volume was added slowly, and meanwhile the cell suspension was mixed gently and uniformly. 7 days after transfection, the cell culture supernatant expressing the protein of interest was centrifuged at 15000 g (high speed) for 10 min, the resulting supernatant was affinity purified using MabSelect SuRe LX (GE, 17547403), and then the protein of interest was eluted with 100 mM sodium acetate (pH 3.0), followed by neutralization with 1 M Tris-HCl, and finally the resulting protein was transferred to PBS buffer by liquid change through an ultrafiltration concentration tube (Millipore, UFC901096).

### Example 4 Identification of physicochemical property of candidate antibody

### 4.1 SDS-PAGE identification of candidate antibody

Preparation of non-reducing solution: The candidate antibody in example 3 and 1 µg of quality control IPI (i.e., Ipilimumab) were added to a 5 × SDS loading buffer and 40 mM of iodoacetamide, heated in a dry bath at 75°C for 10 min, cooled to room temperature, and centrifuged at 12000 rpm for 5 min to collect the supernatant.

Preparation of reducing solution: 2 µg of the candidate antibody of example 3 and the quality control IPI were added to 5× SDS loading buffer and 5 mM DTT, heated under a dry bath at 100°C for 10 min, cooled to room temperature, and centrifuged at 12000 rpm for 5 min and the supernatant was taken.

The supernatant was added to Bis-tris 4%-15% gradient gel (GenScript Biotechnology Co., Ltd.) for gel electrophoresis and the protein bands were visualized by Coomassie Brilliant Blue staining. An EPSON V550 color scanner was used to scan protein gel with stained protein bands (destained using a destaining solution until the gel background became transparent), and the reduced and nonreduced band purity was calculated by ImageJ according to an area normalization method.

The results are shown in Table 1. The band of the candidate antibody in the non-reducing gel was around 104 kD, and the band in the reducing gel was around 60 kD, which was consistent with the expected size. The purity of the antibody molecules P1-3-scFv-LH, P2-3-scFv-LH, P3-1-scFv-LH, 63-scFv-LH, 78-scFv-LH and 79-scFv-LH could be detected by reducing gel, and the antibody purity was greater than 93%.

### 4.2 SEC-HPLC monomer purity identification of candidate antibody

Material preparation: 1. Mobile phase: 150 mmol/L phosphate buffer, pH 7.4; 2. Sample preparation: Both candidate antibody and quality control IPI were diluted to 0.5 mg/mL with mobile phase solution. The flow rate of the Agilent HPLC 1100 column (XBridge BEH SEC 3.5 µm, 7.8 mm I.D. × 30 cm, Waters) was set to 0.8 mL/min, the load volume was 20 µL, and the VWD detector wavelengths were 280 nm and 214 nm. The blank solution (150 mmol/L phosphate buffer), IPI quality control solution and sample solution were loaded in sequence. The percentages of high molecular polymers, antibody monomers and low molecular substances in the sample were calculated according to the area normalization method.

The results are shown in Table 1. The antibody monomer purity of the antibody molecules P1-3-scFv-LH, P2-3-scFv-LH, P3-1-scFv-LH, 63-scFv-LH, 78-scFv-LH and 79-scFv-LH could be detected by SEC-HPLC, and the monomer purity of the antibody molecules was greater than 97%.

**Table 1**

| Protein name | Molecular weight (KD) | Isoelectric point PI | Extinction coefficient | SDS PAGE (%) | SEC-HPLC (%) |
|---|---|---|---|---|---|
| P1-3-scFv-LH | 104.84 | 8.94 | 1.76 | > 95.0 | 100 |
| P2-3-scFv-LH | 104.6 | 8.94 | 1.74 | 94.8 | 100 |
| P3-1-scFv-LH | 104.52 | 8.94 | 1.74 | > 95.0 | 100 |
| P1-1-scFv-LH | 104.52 | 8.94 | 1.74 | N/A | N/A |
| P2-1-scFv-LH | 104.52 | 8.94 | 1.74 | N/A | N/A |
| P2-2-scFv-LH | 104.6 | 8.94 | 1.74 | N/A | N/A |
| P3-4-scFv-LH | 104.6 | 8.94 | 1.74 | N/A | N/A |
| 63-scFv-LH | 104.58 | 8.88 | 1.72 | > 95.0 | 97.75 |
| 78-scFv-LH | 104.76 | 8.8 | 1.74 | > 95.0 | 97.43 |
| 79-scFv-LH | 104.66 | 8.88 | 1.72 | 93.1 | 98.41 |
| 16-scFv-LH | 104.54 | 8.8 | 1.74 | Unable to determine | N/A |
| 23-scFv-LH | 104.52 | 8.95 | 1.72 | Unable to determine | N/A |
| 34-scFv-LH | 104.4 | 8.95 | 1.7 | Unable to determine | N/A |
| 43-scFv-LH | 104.62 | 8.94 | 1.74 | Unable to determine | N/A |
| 67-scFv-LH | 104.46 | 8.95 | 1.72 | 82.3 | N/A |
| H2C-scFv-LH | 104.68 | 8.68 | 1.88 | > 95.0 | 92.7 |

| | | | | | |
|---|---|---|---|---|---|
| Note: "N/A" means that the protein was not purified due to poor drugability of the antibody and SDS-PAGE analysis was not performed; "Unable to determine" means that the antibody purity was poor and there were many miscellaneous bands on SDS-PAGE. | | | | | |

### Example 5 Engineering and physicochemical property identification of anti-CD3 Antibody

Further, molecules P1-3-scFv, P2-3-scFv, P3-1-scFv, 63-scFv, 78-scFv, and 79-scFv having good physicochemical properties obtained by humanization and affinity maturation in example 4 were subjected to combinatorial mutations to obtain engineered molecules P1-3-3-scFv (SEQ ID NO: 14), P2-3-3-scFv (SEQ ID NO: 15), P2-3-5-scFv (SEQ ID NO: 16), 63-P2-3-scFv (SEQ ID NO: 17), 63-P3-1-scFv (SEQ ID NO: 18), 78-P1-3-scFv (SEQ ID NO: 19), 78-P2-3-scFv (SEQ ID NO: 20), 78-P3-1-scFv (SEQ ID NO: 21), 79-P1-3-scFv (SEQ ID NO: 22), 79-P2-3-scFv (SEQ ID NO: 23), and 79-P3-1-scFv (SEQ ID NO: 24).

The obtained molecules were subjected to antibody construction, expression and purification (for specific methods, see Example 3) to obtain antibody molecules P1-3-3-scFv-LH, P2-3-3-scFv-LH, P2-3-5-scFv-LH, 63-P2-3-scFv-LH, 63-P3-1-scFv-LH, 78-P1-3-scFv-LH, 78-P2-3-scFv-LH, 78-P3-1-scFv-LH, 79-P1-3-scFv-LH, 79-P2-3-scFv-LH and 79-P3-1-scFv-LH.

The obtained antibody molecules were subjected to SDS-PAGE identification and SEC-HPLC monomer purity identification. The specific methods are shown in example 4. The experimental results are shown in Table 2.

The purity of the further engineered molecules by SDS-PAGE was greater than 95%, and the monomer purity of the modified molecules identified by SEC-HPLC was greater than 95% except for 79-P3-1-scFv-LH.

**Table 2**

| Protein name | Molecular weight (KD) | Isoelectric point PI | Extinction coefficient | SDS PAGE (%) | SEC-HPLC (%) |
|---|---|---|---|---|---|
| H2C-scFv-LH | 104.68 | 8.68 | 1.88 | > 95.0 | 92.7 |
| P1-3-3-scFv-LH | 104.68 | 8.71 | 1.91 | > 95.0 | 96.88 |
| P2-3-3-scFv-LH | 104.48 | 8.71 | 1.88 | > 95.0 | 95.03 |
| P2-3-5-scFv-LH | 104.46 | 8.71 | 1.88 | > 95.0 | 96.31 |
| 63-P2-3-scFv-LH | 104.44 | 8.61 | 1.86 | > 95.0 | 99.24 |
| 63-P3-1-scFv-LH | 104.36 | 8.61 | 1.86 | > 95.0 | 100.00 |
| 78-P1-3-scFv-LH | 104.86 | 8.47 | 1.91 | > 95.0 | 100.00 |
| 78-P2-3-scFv-LH | 104.64 | 8.48 | 1.88 | > 95.0 | 99.33 |
| 78-P3-1-scFv-LH | 104.58 | 8.48 | 1.88 | > 95.0 | 100.00 |
| 79-P1-3-scFv-LH | 104.76 | 8.6 | 1.88 | > 95.0 | 98.62 |
| 79-P2-3-scFv-LH | 104.52 | 8.61 | 1.85 | > 95.0 | 99.54 |
| 79-P3-1-scFv-LH | 104.46 | 8.61 | 1.86 | > 95.0 | N/A |

### Example 6 Affinity detection of candidate antibody

### 6.1 Detection of affinity activity of engineered antibody based on FACS method

huCD3D-CD3E-CHO-K1 cells or Jurkat cells (human T lymphocytic leukemia cells) in the logarithmic growth phase were collected, centrifuged at 300 g to remove the supernatant; the cells were resuspended in FACS buffer (PBS containing 1% BSA) and counted and the cell suspension density was adjusted to 1 × 10⁶ cells/mL. Subsequently, the cells were added to a 96-well round bottom plate at 100 µL of cell suspension per well, and centrifuged at 300 g to remove the supernatant. Different concentrations of candidate antibody (63-scFv-LH, 78-scFv-LH, 79-scFv-LH, P1-3-scFv-LH, P2-3-scFv-LH, P3-1-scFv-LH, P1-3-3-scFv-LH, P2-3-3-scFv-LH, P2-3-5-scFv-LH, 63-P2-3-scFv-LH, 63-P3-1-scFv-LH, 78-P1-3-scFv-LH, 78-P2-3-scFv-LH, 78-P3-1-scFv-LH, 79-P1-3-scFv-LH, 79-P2-3-scFv-LH, 79-P3-1-scFv-LH, respectively) and control antibody H2C-scFv-LH dilutions were added to the corresponding wells, and the cells were resuspended and incubated at 4°C for 60 min. The incubated cell mixture solution was washed 3 times, then PE-labeled Anti-human-IgG-Fc flow cytometry antibody (Abcam, 98596) was added, resuspended and placed at 4°C for incubation for 30 min. The incubated cell mixture solution was washed 3 times, then 200 µL of FACS buffer was added to resuspend the cells, and detected and analyzed by a flow cytometer (Beckman, CytoFLEX AOO-1-1102). Data were analyzed using PRISMTM (GraphPad Software, San Diego, CA), and EC₅₀ values were calculated.

FACS binding assay results are shown in FIGs. 2A-2D. It can be seen from FIGs. 2A-2B that the upper plateau level and EC₅₀ of binding of P1-3-scFv-LH to huCD3D-CD3E-CHO-K1 cells were both significantly worse than control antibody H2C-scFv-LH. Its affinity, calculated by EC50, was 3.65-fold lower than that of the control antibody, indicating lower affinity; EC₅₀ of binding of P2-3-scFv-LH, 63-scFv-LH, 78-scFv-LH, and 79-scFv-LH to huCD3D-CD3E-CHO-K1 cells was comparable to the control antibody H2C-scFv-LH. The upper plateau level of 63-scFv-LH and 78-scFv-LH was slightly lower than that of the control antibody H2C-scFv-LH (FIG. 2A). EC₅₀ of binding of P3-1-scFv-LH to huCD3D-CD3E-CHO-K1 cells was smaller than that of the control antibody H2C-scFv-LH, and P3-1-scFv-LH exhibited better affinity (FIG. 2B). It can be seen from FIGs. 2C-2D that EC₅₀ of binding of P2-3-3-scFv-LH, P2-3-5-scFv-LH, and 63-P3-1-scFv-LH to Jurkat cells was lower than that of the control antibody H2C-scFv-LH, indicating superior affinity relative to the control antibody H2C-scFv-LH. The binding of 63-P2-3-scFv-LH to Jurkat cells was comparable to that of the control antibody. EC₅₀ of binding of P1-3-3-scFv-LH to Jurkat cells was significantly higher than that of the control antibody H2C-scFv-LH. An approximately two-fold reduction in MFI was observed at concentrations of 1.25 µg/mL, 0.3125 µg/mL, 0.0781 µg/mL, and 0.0195 µg/mL, indicating a lower affinity. Compared to the control antibody, 78-P1-3-scFv-LH exhibited a weak binding to Jurkat cells with an approximately 1000-fold reduction in affinity, indicating an extremely low affinity (FIG. 2C). EC₅₀ of binding of 78-P3-1-scFv-LH to Jurkat cells was lower than that of the control antibody H2C-scFv-LH, but the upper plateau level was lower than that of the control antibody H2C-scFv-LH. The upper plateau levels of binding of 78-P2-3-scFv-LH and 79-P2-3-scFv-LH to Jurkat cells were lower than that of the control antibody H2C-scFv-LH, and the EC₅₀ was comparable to that of the control antibody. EC₅₀ and upper plateau level of binding of 79-P1-3-scFv-LH to Jurkat cells were interior to those of the control antibody H2C-scFv-LH, and the affinity of 79-P1-3-scFv-LH, calculated by EC₅₀, was 3.65-fold lower than that of the control antibody, indicating a lower affinity (FIG. 2D).

### 6.2 Detection of cross-activity of engineered antibody against monkey antigen based on FACS method

CynoCD3E-HEK293 cells in the logarithmic growth phase were collected, centrifuged at 300 g to remove the supernatant; the cells were resuspended in FACS buffer (PBS containing 1% BSA) and counted and the cell suspension density was adjusted to 1 × 10⁶ cells/mL. Subsequently, the cells were added to a 96-well round bottom plate at 100 µL of cell suspension per well, and centrifuged at 300 g to remove the supernatant. Different concentrations of candidate antibody (63-P2-3-scFv-LH, 63-P3-1-scFv-LH, 78-P1-3-scFv-LH, 78-P2-3-scFv-LH, 78-P3-1-scFv-LH, 79-P1-3-scFv-LH, and 79-P2-3-scFv-LH, respectively) and control antibody H2C-scFv-LH dilutions were added to the corresponding wells, wherein the first well of 78-P1-3-scFv-LH had a concentration of 50 µg/mL and 3-fold serial dilution was performed. The first wells of other molecules had a concentration of 5 µg/mL, and 3-fold serial dilution was performed. The cells were resuspended and incubated at 4°C for 60 min. The incubated cell mixture solution was washed 3 times, then PE-labeled Anti-human-IgG-Fc flow cytometry antibody (invitrigen, 12-4998-82) was added, resuspended and placed at 4°C for incubation for 30 min. The incubated cell mixture solution was washed 3 times, then 200 µL of FACS buffer was added to resuspend the cells, and detected and analyzed by a flow cytometer (Beckman, CytoFLEX AOO-1-1102). Data were analyzed using PRISMTM (GraphPad Software, San Diego, CA), and EC₅₀ values were calculated.

The results of the FACS assay are shown in FIG. 3. It can be seen from FIG. 3 that the candidate antibodies 63-P2-3-scFv-LH, 63-P3-1-scFv-LH, 78-P1-3-scFv-LH, 78-P2-3-scFv-LH, 78-P3-1-scFv-LH, 79-P1-3-scFv-LH, and 79-P2-3-scFv-LH all bound to CynoCD3E-HEK293, and exhibited cross activity for binding to monkey CD3, wherein the activity of binding of 63-P3-1-scFv-LH and 78-P3-1-scFv-LH to monkey CD3 overexpressing cells CynoCD3E-HEK293 was slightly higher than that of the control antibody H2C-scFv-LH; the activity of binding of 63-P2-3-scFv-LH, 78-P2-3-scFv-LH, and 79-P2-3-scFv-LH to monkey CD3 overexpressing cells CynoCD3E-HEK293 was comparable to that of the control antibody H2C-scFv-LH; and the activity of binding of 78-P1-3-scFv-LH and 79-P1-3-scFv-LH to monkey CD3 overexpressing cells CynoCD3E-HEK293 was significantly lower than that of the control antibody H2C-scFv-LH.

### 6.3 Detection of affinity activity of candidate antibody based on BLI

In this example, the affinity of the candidate antibody and the control antibody to the huCD3E AA1-27-huFc antigen protein was detected based on the Gator device.

Protein biotinylation: The antigen protein huCD3E AA1-27-huFc was diluted to a final concentration of 1 mg/mL, premixed with biotin and reacted at 4°C for 1 h, and then unreacted free biotin was removed by filtration with an ultrafiltration tube to obtain biotinylated huCD3E AA1-27-huFc (hereinafter referred to as huCD3E-AA1-27-huFc-Biotin).

Q Buffer formulation: 2 g of BSA (IgG-free, purchased from Jackson ImmunoResearch Lab) was weighed and 2 mL of 10% Tween 20 (purchased from Thermo) was measured, and both were added to 1000 mL of 1 × PBS (purchased from Thermo) and mixed well, and the pH was adjusted to 7.40 to prepare a Q Buffer which was filtered, subpackaged, and stored.

Regeneration buffer formulation: 0.38 g of glycine and 4.38 g of sodium chloride were weighed and added into 500 mL of pure water and mixed well, and the pH was adjusted to 2.0 to prepare a sensor regeneration buffer which was filtered, subpackaged, and stored.

Detection: huCD3E-AA1-27-huFc-Biotin was diluted to 75 nM with the Q Buffer, and the candidate antibody or control antibody was subjected to a 2-fold serial dilution with the Q Buffer to 40, 20, 10, 5, 2.5, 1.25, 0.62, and 0 nM, respectively. In the dark, the sensor was pre-wetted with Q Buffer (SA Probes, ProbeLife). After 10 min, the sample plate (Greiner, 655209) was tested. After the test was correct, the preset procedure was followed. First, antigen binding was performed for 600 s. After the binding was completed and equilibration was continued in Q Buffer for 30 s, the sensor bound to the antigen huCD3E-AA1-27-huFc-Biotin was transferred to different concentrations of antibody dilutions for binding for 120 s, and then transferred to Q Buffer for dissociation for 180 s. Finally, K_{D}, Ka and Kd were obtained by fitting the binding and dissociation data of different concentrations of antigens and antibodies.

Results are as shown in FIGs. 4A-4Q and Table 3. P1-3-scFv-LH, P1-3-3-scFv-LH, 78-P1-3-scFv and 79-P1-3-scFv exhibited significantly lower affinity to the antigen protein than the parent antibody. In particular, the K_{D} value of 78-P1-3-scFv-LH increased from 10⁻¹⁰ M to 10⁻⁸ M. However, the affinity of engineered antibody 78-P2-3-scFv-LH to antigen protein was higher than that of parent antibody.

**Table 3**

| Protein name | huCD3E AA1-27-huFc-Biotin | | | | |
|---|---|---|---|---|---|
| | K_{D} (M) | ka (1/Ms) | kd (1/s) | R² | Rmax (nm) |
| H2C-scFv-LH | 3.18E-10 | 1.03E+06 | 3.27E-04 | 0.992 | 0.242 |
| 63-scFv-LH | 3.58E-10 | 1.19E+06 | 4.28E-04 | 0.994 | 0.222 |
| 78-scFv-LH | 1.81E-10 | 1.70E+06 | 3.08E-04 | 0.988 | 0.235 |
| 79-scFv-LH | 2.22E-10 | 8.26E+05 | 1.84E-04 | 0.994 | 0.367 |
| P1-3-scFv-LH | 5.86E-09 | 6.35E+05 | 3.72E-03 | 0.99 | 0.249 |
| P2-3-scFv-LH | 8.86E-10 | 7.79E+05 | 6.90E-04 | 0.994 | 0.29 |
| P3-1-scFv-LH | 4.99E-10 | 1.17E+06 | 5.84E-04 | 0.989 | 0.282 |
| P1-3-3-scFv-LH | 3.94E-09 | 6.02E+05 | 2.37E-03 | 0.995 | 0.201 |
| P2-3-3-scFv-LH | 3.66E-10 | 1.24E+06 | 4.53E-04 | 0.992 | 0.275 |
| P2-3-5-scFv-LH | 3.32E-10 | 1.23E+06 | 4.10E-04 | 0.995 | 0.27 |
| 63-P2-3-scFv-LH | 3.04E-10 | 1.60E+06 | 4.86E-04 | 0.996 | 0.228 |
| 63-P3-1-scFv-LH | 1.36E-10 | 1.66E+06 | 2.26E-04 | 0.993 | 0.252 |
| 78-P1-3-scFv-LH | 3.31E-08 | 4.31E+05 | 1.43E-02 | 0.976 | 0.137 |
| 78-P2-3-scFv-LH | 4.90E-11 | 1.29E+06 | 6.33E-05 | 0.992 | 0.227 |
| 78-P3-1-scFv-LH | 2.16E-10 | 2.01E+06 | 4.34E-04 | 0.995 | 0.257 |
| 79-P1-3-scFv-LH | 4.69E-09 | 6.18E+05 | 2.90E-03 | 0.988 | 0.152 |
| 79-P2-3-scFv-LH | 5.06E-10 | 8.07E+05 | 4.08E-04 | 0.995 | 0.24 |
| 79-P3-1-scFv-LH | 3.84E-10 | 1.23E+06 | 4.71E-04 | 0.996 | 0.246 |

### 6.4 Detection of affinity activity of candidate antibody based on SPR

In this example, the affinity of the candidate antibody to the huCD3E AA1-27-huFc antigen protein was detected based on the Biacore device.

The specific method was as follows:
Protein coupling: The huCD3E-AA1-27-huFc protein produced in example 1.2 was diluted to 5.8 µg/mL with an NaAc buffer at pH 4.5. The flow rate was set to 10 µL/min. The chip was activated with a mixture of 1-ethyl-(3-dimethylaminopropyl)carbodiimide (EDC) and N-hydroxysuccinimide (NHS) for the default time of 420 s. The antigen protein huCD3E AA1-27-huFc was immobilized to a level of approximately 75 RU using a coupling mode with a preset coupling amount. The activated groups not bound to the test sample were blocked with ethanolamine.

Sample test condition: A PBS buffer (pH 7.4) containing 0.05% Tween-20 was used as a running buffer, and the running buffer was used as a control antibody test sample. A series of antibody concentrations (4 nM, 20 nM) were set. During sample analysis, the flow rate was set to 30 µL/min, the binding time was 180 s, and the dissociation time was 300 s. After dissociation was completed, regeneration was performed with 10 mM Gly-HCl (pH 2.0) for 20 s to completely remove antibody bound to the antigen.

Parameter fitting: The experiment was run in multiple cycles, with the response signal plotted against analysis time on the abscissa and response value on the ordinate. After dual-reference subtraction, the obtained data were fitted by BIAcore T200 analysis software. The fitting model used was a 1 : 1 Langmuir binding model, so as to determine affinity indicators such as the binding dissociation constant.

Results are as shown in FIGs. 5A-5H and Table 4. The affinity of 63-P2-3-scFv-LH, 63-P3-1-scFv-LH, 78-P2-3-scFv-LH, 78-P3-1-scFv-LH, and 79-P2-3-scFv-LH to the antigen protein was comparable to that of the parent antibody. The affinity of the engineered antibodies 78-P1-3-scFv-LH and 79-P1-3-scFv-LH to the antigen protein was significantly lower than that of the parent antibody. The Koff of 78-P1-3-scFv-LH and 79-P1-3-scFv-LH was reduced to 1.77E-2 and 8.23E-3, respectively. The binding dissociation constants K_{D} of 78-P1-3-scFv-LH and 79-P1-3-scFv-LH to the antigen protein was 8.71E-09 and 1.81E-09, respectively.

**Table 4**

| Protein name | huCD3E AA1-27-huFc | | | | |
|---|---|---|---|---|---|
| | K_{D} (M) | ka(1/Ms) | kd(1/s) | Rmax (nm) | Chi2 |
| H2C-scFv-LH | 5.96E-11 | 2.77E+06 | 1.65E-04 | 83.75 | 0.0321 |
| 63-P2-3-scFv-LH | 6.51E-11 | 2.27E+06 | 1.48E-04 | 73.87 | 0.019 |
| 63-P3-1-scFv-LH | 5.69E-11 | 2.43E+06 | 1.38E-04 | 80.47 | 0.0216 |
| 78-P1-3-scFv-LH | 8.71E-09 | 2.03E+06 | 1.77E-02 | 22.11 | 0.358 |
| 78-P2-3-scFv-LH | 8.86E-11 | 1.26E+06 | 1.12E-04 | 72.02 | 0.029 |
| 78-P3-1-scFv-LH | 4.85E-11 | 1.93E+06 | 9.38E-05 | 73.8 | 0.0154 |
| 79-P1-3-scFv-LH | 1.81E-09 | 4.54E+06 | 8.23E-03 | 30.44 | 0.114 |
| 79-P2-3-scFv-LH | 7.04E-11 | 1.67E+06 | 1.18E-04 | 79.79 | 0.12 |

### Example 7 Detection of T cell activating activity of candidate antibody based on reporter method

In this example, a luciferase reporter gene system was used to detect the activity of the antibody in activating the NF-κB signaling pathway before and after engineering. The anti-CD3 antibody bound to CD3 and activated its downstream NF-κB signal transduction, thereby inducing the expression of luciferase. Stimulation with antibodies at different concentration gradients would produce a fluorescence reading curve that was dependent on antibody concentration, thereby evaluating the activating activity of the antibody. In this example, a luciferase reporter gene system was used to detect the activity of the antibody before and after engineering in activating the NF-κB signaling pathway. The specific method was as follows:
Serially diluted candidate antibody and control antibody were added to wells of a 96-well white opaque frame/clear bottom cell culture plate at 100 µL/well, and incubated overnight at 4°C. On the next day, NF-κB-Jurkat cells in the logarithmic growth phase were collected and centrifuged at 300 g to remove the supernatant. The cells were resuspended in the FACS buffer (PBS containing 1% BSA) and counted, and a cell suspension density was adjusted to 1 × 10⁶ cells/mL. The 96-well white opaque frame/clear bottom cell culture plate was washed 3 times with PBS, and the cell suspension was added at 100 µL/well, and incubated in an incubator at 37°C for 6 h. 50 µL of Bright-Lite (vazyme, Cat. No. DD1204-03) was added to each well; the mixture was incubated in the dark for 10 min; and the fluorescence signal was detected. Data were analyzed using PRISMTM (GraphPad Software, San Diego, CA).

The results of the activating activity assay are shown in FIGs. 6A-6F. The results showed that: Compared with the control antibody H2C-scFv-LH, P2-3-scFv-LH exhibited a higher activation upper plateau level and better T cell activating activity. P1-3-scFv-LH exhibited a higher EC₅₀ and a lower upper plateau level than the control antibody, and the affinity of P1-3-scFv-LH, calculated by EC₅₀, was approximately 7-fold lower than that of the control antibody, indicating a lower T cell activating activity (FIG. 6A). P3-1-scFv-LH exhibited a higher upper plateau level of activation than the control antibody, with higher T cell activating activity (FIG. 6B); 63-scFv-LH, 78-scFv-LH exhibited lower activating activity compared to 79-scFv-LH (FIG. 6C); The EC₅₀ of activation of cells by P1-3-3-scFv-LH and P2-3-5-scFv-LH was higher than that of the control antibody H2C-scFv-LH, indicating a lower T cell activating activity, wherein compared with the control antibody H2C-scFv-LH, the activating activity of P1-3-3-scFv-LH was reduced by 7.69-fold, and the activating activity of P2-3-5-scFv-LH was reduced by 6.86-fold (FIG. 6D); 63-P2-3-scFv-LH exhibited a lower activation upper plateau level than the control antibody H2C-scFv-LH. The activating activity (EC₅₀) of 63-P3-1-scFv-LH was about 2-fold lower than that of the control antibody H2C-scFv-LH, indicating a lower T cell activating activity. Compared with the control antibody H2C-scFv-LH, 78-P2-3-scFv-LH exhibited a lower activation upper plateau level and a higher activation EC₅₀, and exhibited a lower T cell activating activity. 78-P1-3-scFv-LH exhibited extremely weak T cell activating activity (FIG. 6E); 79-P2-3-scFv-LH exhibited an activation upper plateau level and EC₅₀ comparable to the control antibody, and an activating activity comparable to the control antibody. The activating activity (EC₅₀) of 78-P3-1-scFv-LH was 2.62-fold lower than that of the control antibody H2C-scFv-LH, indicating a lower T cell activating activity. 79-P1-3-scFv-LH exhibited lower activating activity than the control antibody H2C-scFv-LH, and the activation fluorescence intensity at the highest concentration of 1000 µg/mL was 3.7-fold lower than that of the control antibody H2C-scFv-LH.

Studies have shown that lower T cell activating activity can avoid over-activation of T cells and reduce the probability of abnormal cytokine release. In this example, the 78-P2-3-scFv-LH antibody molecule reduced T cell activating activity while retaining a high affinity. It could be predicted that the bispecific antibody containing the antibody sequence could avoid over-activation of T cells and reduce the probability of abnormal cytokine release while improving the affinity of CD3 bispecific antibody to CD3 on the surface of T cells, narrowing the distance between tumor cells and T cells, and then promoting the binding of surface receptors such as TCR on the surface of T cells and pMHC on the surface of tumors, and inducing T cells to secrete perforin and granzymes to kill tumor cells.

### Example 8 Cytokine secretion assay of candidate antibody

In this example, the ability of antibodies before and after engineering to induce cytokine IFN-γ secretion was detected by ELISA. The binding of anti-CD3 antibody to CD3 on T cells could activate T cells to induce the secretion of cytokines such as IL2, IL6, IL10, IFN-γ, and TNF-α. Stimulation with anti-CD3 antibodies at different concentration gradients could obtain antibody concentration-dependent cytokine secretion amounts, and thus the T cell activating activity of anti-CD3 antibodies could be evaluated.

The specific method was as follows: first, the candidate antibody was serially diluted with an initial concentration of 8 µg/mL, added to a 96-well plate (Corning, 3370), and incubated at 4°C overnight; On the next day, the plate was washed 3 times with pre-cooled PBS, and peripheral blood mononuclear cells (PBMC) were adjusted to 1 × 10⁶ cells/mL, 100 µL of PBMC suspension and 100 µL of CD28 antibody (Biolegend, 302934) at a concentration of 1 µg/mL were added to each well. Then the 96-well plate was placed in a 37°C cell culture incubator containing 5% CO₂ and incubated for 48 h. The supernatant was removed by centrifugation and detection was performed by ELISA. Data were analyzed using PRISMTM (GraphPad Software, San Diego, CA).

The results are shown in FIGs. 7A-7E. The results showed that: 63-scFv-LH at concentrations of 8 µg/mL and 1.28 µg/mL induced higher IFN-γ secretion than the control antibody H2C-scFv-LH, and 78-scFv-LH at concentrations of 8 µg/mL, 1.28 µg/mL, and 0.64 µg/mL induced higher IFN-γ secretion than the control antibody H2C-scFv-LH (FIG. 7A); 79-scFv-LH at concentrations of 8 µg/mL, 3.20 µg/mL, 1.28 µg/mL, 0.64 µg/mL and 0.32 µg/mL induced higher IFN-γ secretion than the control antibody H2C-scFv-LH, demonstrating that its ability to induce IFN-γ secretion was significantly higher than that of the control antibody H2C-scFv-LH. P1-3-scFv-LH at concentrations of 8 µg/mL, 3.20 µg/mL, 1.28 µg/mL and 0.64 µg/mL induced lower IFN-γ secretion than the control H2C-scFv-LH, demonstrating that its ability to induce IFN-γ secretion was significantly lower than that of the control antibody H2C-scFv-LH (FIG. 7B); P2-3-scFv-LH and P3-1-scFv at 8 µg/mL, 3.20 µg/mL, 1.28 µg/mL, and 0.64 µg/mL concentrations induced higher IFN-γ secretion than the control antibody H2C-scFv-LH, demonstrating their significantly improved ability to induce IFN-γ secretion compared with the control antibody H2C-scFv-LH (FIG. 7C). 78-P1-3-scFv-LH at concentrations of 8 µg/mL and 3.20 µg/mL induced significantly lower IFN-γ secretion than the control antibody H2C-scFv-LH, and hardly induced IFN-γ secretion at lower concentrations, demonstrating its significantly reduced ability to induce IFN-γ secretion compared with the control antibody H2C-scFv-LH. The ability of 78-P2-3-scFv-LH to induce IFN-γ secretion was comparable to that of the control antibody H2C-scFv-LH (FIG. 7D). The ability of 79-P1-3-scFv-LH to induce IFN-γ secretion was significantly lower than that of the control antibody H2C-scFv-LH, and 79-P1-3-scFv-LH at 8 µg/mL, 3.20 µg/mL, 0.64 µg/mL, and 0.32 µg/mL concentrations induced lower IFN-γ secretion than the control antibody H2C-scFv-LH (FIG. 7E).

Studies have shown that low-affinity CD3 antibodies could effectively increase the distribution of bispecific antibodies in tumor tissues, reduce the distribution of bispecific antibodies in other secondary lymphoid tissues, reduce the probability of abnormal cytokine release, and thus reduce the toxicity of CD3 bispecific antibodies. In this example, P1-3-scFv-LH, 79-P1-3-scFv-LH, and 78-P1-3-scFv-LH induced significantly reduced cytokine IFN-γ secretion, and it is predicted that the bispecific antibody comprising the sequences thereof has higher safety.

While exemplary embodiments of the present invention are described above, it will be understood by those skilled in the art that these disclosures are merely exemplary and that various other substitutions, adaptations, and modifications may be made within the scope of the present invention. Accordingly, the present invention is not limited to the specific embodiments recited herein.

### Exemplary sequence

| SEQ ID NO | Sequence description | Sequence information |
|---|---|---|
| 1 | H2C, 63-scFv, 79-scFv Heavy chain variable region (VH) | EVQLVESGGGLVQPGGSLKLSCAASGFTFNKYAMNWV RQAPGKGLEWVARIRSKYNNYATYYADSVKDRFTISR DDSKNTAYLQMNNLKTEDTAVYYCVRHGNFGNSYISY WAYWGQGTLVTVSS |
| 2 | H2C, P3-1-scFv Light chain variable region (VL) | QTVVTQEPSLTVSPGGTVTLTCGSSTGAVTSGYYPNWV QQKPGQAPRGLIGGTKFLAPGTPARFSGSLLGGKAALT LSGVQPEDEAEYYCALWYSNRWVFGGGTKLTVL |
| 3 | CD3E Extracellular domain AA1-27 | QDGNEEMGGITQTPYKVSISGTTVILT |
| 4 | Human IgG1 Fc | THTCPPCPAPELLGGPSVFLFPPKPKDTLMISRTPEVTCV VVDVSHEDPQVKFNWYVDGVQVHNAKTKPREQQYNS TYRVVSVLTVLHQNWLDGKEYKCKVSNKALPAPIEKTI SKAKGQPREPQVYTLPPSREEMTKNQVSLTCLVKGFYP SDIAVEWESNGQPENNYKTTPPVLDSDGSFFLYSKLTV DKSRWQQGNVFSCSVMHEALHNHYTQKSLSLSPGK |
| 5 | CD3D | THTCPPCPAPELLGGPSVFLFPPKPKDTLMISRTPEVTCV VVDVSHEDPQVKFNWYVDGVQVHNAKTKPREQQYNS TYRVVSVLTVLHQNWLDGKEYKCKVSNKALPAPIEKTI SKAKGQPREPQVYTLPPSREEMTKNQVSLTCLVKGFYP SDIAVEWESNGQPENNYKTTPPVLDSDGSFFLYSKLTV DKSRWQQGNVFSCSVMHEALHNHYTQKSLSLSPGK MEHSTFLSGLVLATLLSQVSPFKIPIEELEDRVFVNCNTS ITWVEGTVGTLLSDITRLDLGKRILDPRGIYRCNGTDIY KDKESTVQVHYRMCQSCVELDPATVAGIIVTDVIATLL LALGVFCFAGHETGRLSGAADTQALLRNDQVYQPLRD RDDAQYSHLGGNWARNK MQSGTHWRVLGLCLLSVGVWGQDGNEEMGGITQTPY KVSISGTTVILTCPQYPGSEILWQHNDKNIGGDEDDKNI GSDEDHLSLKEFSELEQSGYYVCYPRGSKPEDANFYLY LRARVCENCMEMDVMSVATIVIVDICITGGLLLLVYYW SKNRKAKAKPVTRGAGAGGRQRGQNKERPPPVPNPDY EPIRKGQRDLYSGLNQRRI QTVVTQEPSLTVSPGGTVTLTCGSSTGAVTSGYYPNWV QQKPGQAPRGLIGGTKFLAPGTPARFSGSLLGGKAALT LSGVQPEDEAEYYCALWYSNRWVFGGGTKLTVLGGG GSGGGGSGGGGSEVQLVESGGGLVQPGGSLKLSCAAS GFTFNKYAMNWVRQAPGKGLEWVARIRSKYNNYATY |
| 6 | CD3E | MQSGTHWRVLGLCLLSVGVWGQDGNEEMGGITQTPY KVSISGTTVILTCPQYPGSEILWQHNDKNIGGDEDDKNI GSDEDHLSLKEFSELEQSGYYVCYPRGSKPEDANFYLY LRARVCENCMEMDVMSVATIVIVDICITGGLLLLVYYW SKNRKAKAKPVTRGAGAGGRQRGQNKERPPPVPNPDY EPIRKGQRDLYSGLNQRRI |
| 7 | H2C-ScFv | QTVVTQEPSLTVSPGGTVTLTCGSSTGAVTSGYYPNWV QQKPGQAPRGLIGGTKFLAPGTPARFSGSLLGGKAALT LSGVQPEDEAEYYCALWYSNRWVFGGGTKLTVLGGG GSGGGGSGGGGSEVQLVESGGGLVQPGGSLKLSCAAS GFTFNKYAMNWVRQAPGKGLEWVARIRSKYNNYATY |
| | | YADSVKDRFTISRDDSKNTAYLQMNNLKTEDTAVYYC VRHGNFGNSYISYWAYWGQGTLVTVSS |
| 8 | P1-3-ScFv | QTVVTQEPSLTVSPGGTVTLTCGSSTGAVTSGYYPNWV QQKPGQAPRALIYGTKFLAPGTPARFSGSLLGGKAALT LSGVQPEDEAEYYCALWYSNRWVFGGGTKLTVLGGG GSGGGGSGGGGSEVQLVESGGGLVQPGGSLKLSCAAS GFTFNKYAMNWVRQAPGKGLEWVGRIRSKYNNYATY YADSVKDRFTISRDDSKNTAYLQMNNLKTEDTAVYYC VRHGNFGNSYISYWAYWGQGTTVTVSS |
| 9 | P2-3-ScFv | QTVVTQEPSLTVSPGGTVTLTCGSSTGAVTSGYYPNWV QQKPGQAPRALIGGTKFLAPGTPARFSGSLLGGKAALT LSGVQPEDEAEYYCALWYSNRWVFGGGTKLTVLGGG GSGGGGSGGGGSEVQLVESGGGLVQPGGSLKLSCAAS GFTFSKYAMNWVRQAPGKGLEWVARIRSKYNNYATY YADSVKDRFTISRDDSKNTAYLQMNNLKTEDTAVYYC VRHGNFGNSYISYWAYWGQGTTVTVSS |
| 10 | P3-1-ScFv | QTVVTQEPSLTVSPGGTVTLTCGSSTGAVTSGYYPNWV QQKPGQAPRGLIGGTKFLAPGTPARFSGSLLGGKAALT LSGVQPEDEAEYYCALWYSNRWVFGGGTKLTVLGGG GSGGGGSGGGGSEVQLVESGGGLVQPGGSLKLSCAAS GFTFSKYAMNWVRQASGKGLEWVGRIRSKYNNYATY YADSVKDRFTISRDDSKNTAYLQMNNLKTEDTAVYYC VRHGNFGNSYISYWAYWGQGTTVTVSS |
| 11 | 63-ScFv | QTVVTQEPSLTVSPGGTVTLTCGSSTGAVTSGYYPNWV QQKPGQAPRGLIGGTKFLAPGTPARFSGSLLGGKAALT LSGVQPEDEAEYYCALWVDNRWVFGGGTKLTVLGGG GSGGGGSGGGGSEVQLVESGGGLVQPGGSLKLSCAAS GFTFNKYAMNWVRQAPGKGLEWVARIRSKYNNYATY YADSVKDRFTISRDDSKNTAYLQMNNLKTEDTAVYYC VRHGNFGNSYISYWAYWGQGTLVTVSS |
| 12 | 78-ScFv | QTVVTQEPSLTVSPGGTVTLTCGSSTGAVTSGYYPNWV QQKPGQAPRGLIGGTHFLAPGTPARFSGSLLGGKAALT LSGVQPEDEAEYYCALWYSNRWVFGGGTKLTVLGGG GSGGGGSGGGGSEVQLVESGGGLVQPGGSLKLSCAAS GFTFPKYAMNWVRQAPGKGLEWVARIRSKYNNYETY YADSVKDRFTISRDDSKNTAYLQMNNLKTEDTAVYYC VRHGNFGNSYISYWAYWGQGTLVTVSS |
| 13 | 79-ScFv | QTVVTQEPSLTVSPGGTVTLTCGSSTGAVTSGYYPNWV QQKPGQAPRGLIGGTKFLAPGTPARFSGSLLGGKAALT LSGVQPEDEAEYYCALWQENRWVFGGGTKLTVLGGG GSGGGGSGGGGSEVQLVESGGGLVQPGGSLKLSCAAS GFTFNKYAMNWVRQAPGKGLEWVARIRSKYNNYATY YADSVKDRFTISRDDSKNTAYLQMNNLKTEDTAVYYC VRHGNFGNSYISYWAYWGQGTLVTVSS |
| 14 | P1-3-3-scFv | QTVVTQEPSLTVSPGGTVTLTCGSSTGAVTSGYYPNWV QQKPGQAPRALIYGTKFLAPGTPARFSGSLLGGKAALT LSGVQPEDEAEYYCALWYSNRWVFGGGTKLTVLGGG GSGGGGSGGGGSEVQLVESGGGLVQPGGSLKLSCAAS GFTFSKYAMNWVRQASGKGLEWVGRIRSKYNNYATY YADSVKDRFTISRDDSKNTAYLQMNNLKTEDTAVYYC VRHGNFGNSYISYWAYWGQGTTVTVSS |
| 15 | P2-3-3-scFv | QTVVTQEPSLTVSPGGTVTLTCGSSTGAVTSGYYPNWV QQKPGQAPRALIGGTKFLAPGTPARFSGSLLGGKAALT LSGVQPEDEAEYYCALWYSNRWVFGGGTKLTVLGGG GSGGGGSGGGGSEVQLVESGGGLVQPGGSLKLSCAAS GFTFSKYAMNWVRQAPGKGLEWVGRIRSKYNNYATY YADSVKDRFTISRDDSKNTAYLQMNNLKTEDTAVYYC VRHGNFGNSYISYWAYWGQGTTVTVSS |
| 16 | P2-3-5-scFv | QTVVTQEPSLTVSPGGTVTLTCGSSTGAVTSGYYPNWV QQKPGQAPRALIGGTKFLAPGTPARFSGSLLGGKAALT LSGVQPEDEAEYYCALWYSNRWVFGGGTKLTVLGGG GSGGGGSGGGGSEVQLVESGGGLVQPGGSLKLSCAAS GFTFSKYAMNWVRQASGKGLEWVGRIRSKYNNYATY YADSVKDRFTISRDDSKNTAYLQMNNLKTEDTAVYYC VRHGNFGNSYISYWAYWGQGTTVTVSS |
| 17 | 63-P2-3-scFv | QTVVTQEPSLTVSPGGTVTLTCGSSTGAVTSGYYPNWV QQKPGQAPRALIGGTKFLAPGTPARFSGSLLGGKAALT LSGVQPEDEAEYYCALWVDNRWVFGGGTKLTVLGGG GSGGGGSGGGGSEVQLVESGGGLVQPGGSLKLSCAAS GFTFSKYAMNWVRQAPGKGLEWVARIRSKYNNYATY YADSVKDRFTISRDDSKNTAYLQMNNLKTEDTAVYYC VRHGNFGNSYISYWAYWGQGTTVTVSS |
| 18 | 63-P3-1-scFv | QTVVTQEPSLTVSPGGTVTLTCGSSTGAVTSGYYPNWV QQKPGQAPRGLIGGTKFLAPGTPARFSGSLLGGKAALT LSGVQPEDEAEYYCALWVDNRWVFGGGTKLTVLGGG |
| | | GSGGGGSGGGGSEVQLVESGGGLVQPGGSLKLSCAAS GFTFSKYAMNWVRQASGKGLEWVGRIRSKYNNYATY YADSVKDRFTISRDDSKNTAYLQMNNLKTEDTAVYYC VRHGNFGNSYISYWAYWGQGTTVTVSS |
| 19 | 78-P1-3-scFv | QTVVTQEPSLTVSPGGTVTLTCGSSTGAVTSGYYPNWV QQKPGQAPRALIYGTHFLAPGTPARFSGSLLGGKAALT LSGVQPEDEAEYYCALWYSNRWVFGGGTKLTVLGGG GSGGGGSGGGGSEVQLVESGGGLVQPGGSLKLSCAAS GFTFPKYAMNWVRQAPGKGLEWVGRIRSKYNNYETY YADSVKDRFTISRDDSKNTAYLQMNNLKTEDTAVYYC VRHGNFGNSYISYWAYWGQGTTVTVSS |
| 20 | 78-P2-3-scFv | QTVVTQEPSLTVSPGGTVTLTCGSSTGAVTSGYYPNWV QQKPGQAPRALIGGTHFLAPGTPARFSGSLLGGKAALT LSGVQPEDEAEYYCALWYSNRWVFGGGTKLTVLGGG GSGGGGSGGGGSEVQLVESGGGLVQPGGSLKLSCAAS GFTFSKYAMNWVRQAPGKGLEWVARIRSKYNNYETY YADSVKDRFTISRDDSKNTAYLQMNNLKTEDTAVYYC VRHGNFGNSYISYWAYWGQGTTVTVSS |
| 21 | 78-P3-1-scFv | QTVVTQEPSLTVSPGGTVTLTCGSSTGAVTSGYYPNWV QQKPGQAPRGLIGGTHFLAPGTPARFSGSLLGGKAALT LSGVQPEDEAEYYCALWYSNRWVFGGGTKLTVLGGG GSGGGGSGGGGSEVQLVESGGGLVQPGGSLKLSCAAS GFTFSKYAMNWVRQASGKGLEWVGRIRSKYNNYETY YADSVKDRFTISRDDSKNTAYLQMNNLKTEDTAVYYC VRHGNFGNSYISYWAYWGQGTTVTVSS |
| 22 | 79-P1-3-scFv | QTVVTQEPSLTVSPGGTVTLTCGSSTGAVTSGYYPNWV QQKPGQAPRALIYGTKFLAPGTPARFSGSLLGGKAALT LSGVQPEDEAEYYCALWQENRWVFGGGTKLTVLGGG GSGGGGSGGGGSEVQLVESGGGLVQPGGSLKLSCAAS GFTFNKYAMNWVRQAPGKGLEWVGRIRSKYNNYATY YADSVKDRFTISRDDSKNTAYLQMNNLKTEDTAVYYC VRHGNFGNSYISYWAYWGQGTTVTVSS |
| 23 | 79-P2-3-scFv | QTVVTQEPSLTVSPGGTVTLTCGSSTGAVTSGYYPNWV QQKPGQAPRALIGGTKFLAPGTPARFSGSLLGGKAALT LSGVQPEDEAEYYCALWQENRWVFGGGTKLTVLGGG GSGGGGSGGGGSEVQLVESGGGLVQPGGSLKLSCAAS GFTFSKYAMNWVRQAPGKGLEWVARIRSKYNNYATY |
| | | YADSVKDRFTISRDDSKNTAYLQMNNLKTEDTAVYYC VRHGNFGNSYISYWAYWGQGTTVTVSS |
| 24 | 79-P3-1-scFv | QTVVTQEPSLTVSPGGTVTLTCGSSTGAVTSGYYPNWV QQKPGQAPRGLIGGTKFLAPGTPARFSGSLLGGKAALT LSGVQPEDEAEYYCALWQENRWVFGGGTKLTVLGGG GSGGGGSGGGGSEVQLVESGGGLVQPGGSLKLSCAAS GFTFSKYAMNWVRQASGKGLEWVGRIRSKYNNYATY YADSVKDRFTISRDDSKNTAYLQMNNLKTEDTAVYYC VRHGNFGNSYISYWAYWGQGTTVTVSS |
| 25 | Human IgG1 constant region Fc | ESKYGPPCPPCPAPEFLGGPSVFLFPPKPKDTLMISRTPE VTCVVVDVSQEDPEVQFNWYVDGVEVHNAKTKPREE QFNSTYRVVSVLTVLHQDWLNGKEYKCKVSNKGLPSSI EKTISKAKGQPREPQVYTLPPSQEEMTKNQVSLTCLVK GFYPSDIAVEWESNGQPENNYKTTPPVLDSDGSFFLYSR LTVDKSRWQEGNVFSCSVMHEALHNHYTQKSLSLSLG K |
| 26 | P1-3-scFv, 79-P1-3-scFv VH | EVQLVESGGGLVQPGGSLKLSCAASGFTFNKYAMNWV RQAPGKGLEWVGRIRSKYNNYATYYADSVKDRFTISR DDSKNTAYLQMNNLKTEDTAVYYCVRHGNFGNSYISY WAYWGQGTTVTVSS |
| 27 | P1-3-scFv, P1-3-3-scFv VL | QTVVTQEPSLTVSPGGTVTLTCGSSTGAVTSGYYPNWV QQKPGQAPRALIYGTKFLAPGTPARFSGSLLGGKAALT LSGVQPEDEAEYYCALWYSNRWVFGGGTKLTVL |
| 28 | P2-3-scFv, 63-P2-3-scFv, 79-P2-3-scFv VH | EVQLVESGGGLVQPGGSLKLSCAASGFTFSKYAMNWV RQAPGKGLEWVARIRSKYNNYATYYADSVKDRFTISR DDSKNTAYLQMNNLKTEDTAVYYCVRHGNFGNSYISY WAYWGQGTTVTVSS |
| 29 | P2-3-scFv, P2-3-3-scFv, P2-3-5-scFv VL | QTVVTQEPSLTVSPGGTVTLTCGSSTGAVTSGYYPNWV QQKPGQAPRALIGGTKFLAPGTPARFSGSLLGGKAALT LSGVQPEDEAEYYCALWYSNRWVFGGGTKLTVL |
| 30 | P3-1-scFv, P1-3-3-scFv, P2-3-5-scFv, 63-P3-1-scFv, 79-P3-1-scFv VH | EVQLVESGGGLVQPGGSLKLSCAASGFTFSKYAMNWV RQASGKGLEWVGRIRSKYNNYATYYADSVKDRFTISR DDSKNTAYLQMNNLKTEDTAVYYCVRHGNFGNSYISY WAYWGQGTTVTVSS |
| 31 | 63-scFv, 63-P3-1-scFv VL | QTVVTQEPSLTVSPGGTVTLTCGSSTGAVTSGYYPNWV QQKPGQAPRGLIGGTKFLAPGTPARFSGSLLGGKAALT LSGVQPEDEAEYYCALWVDNRWVFGGGTKLTVL |
| 32 | 78-scFv VH | EVQLVESGGGLVQPGGSLKLSCAASGFTFPKYAMNWV RQAPGKGLEWVARIRSKYNNYETYYADSVKDRFTISRD DSKNTAYLQMNNLKTEDTAVYYCVRHGNFGNSYISYW AYWGQGTLVTVSS |
| 33 | 78-scFv, 78-P3-1-scFv VL | QTVVTQEPSLTVSPGGTVTLTCGSSTGAVTSGYYPNWV QQKPGQAPRGLIGGTHFLAPGTPARFSGSLLGGKAALT LSGVQPEDEAEYYCALWYSNRWVFGGGTKLTVL |
| 34 | 79-scFv VL | QTVVTQEPSLTVSPGGTVTLTCGSSTGAVTSGYYPNWV QQKPGQAPRGLIGGTKFLAPGTPARFSGSLLGGKAALT LSGVQPEDEAEYYCALWQENRWVFGGGTKLTVL |
| 35 | P2-3-3-scFv VH | EVQLVESGGGLVQPGGSLKLSCAASGFTFSKYAMNWV RQAPGKGLEWVGRIRSKYNNYATYYADSVKDRFTISR DDSKNTAYLQMNNLKTEDTAVYYCVRHGNFGNSYISY WAYWGQGTTVTVSS |
| 36 | 63-P2-3-scFv VL | QTVVTQEPSLTVSPGGTVTLTCGSSTGAVTSGYYPNWV QQKPGQAPRALIGGTKFLAPGTPARFSGSLLGGKAALT LSGVQPEDEAEYYCALWVDNRWVFGGGTKLTVL |
| 37 | 78-P1-3-scFv VH | EVQLVESGGGLVQPGGSLKLSCAASGFTFPKYAMNWV RQAPGKGLEWVGRIRSKYNNYETYYADSVKDRFTISRD DSKNTAYLQMNNLKTEDTAVYYCVRHGNFGNSYISYW AYWGQGTTVTVSS |
| 38 | 78-P1-3-scFv VL | QTVVTQEPSLTVSPGGTVTLTCGSSTGAVTSGYYPNWV QQKPGQAPRALIYGTHFLAPGTPARFSGSLLGGKAALT LSGVQPEDEAEYYCALWYSNRWVFGGGTKLTVL |
| 39 | 78-P2-3-scFv VH | EVQLVESGGGLVQPGGSLKLSCAASGFTFSKYAMNWV RQAPGKGLEWVARIRSKYNNYETYYADSVKDRFTISRD DSKNTAYLQMNNLKTEDTAVYYCVRHGNFGNSYISYW AYWGQGTTVTVSS |
| 40 | 78-P2-3-scFv VL | QTVVTQEPSLTVSPGGTVTLTCGSSTGAVTSGYYPNWV QQKPGQAPRALIGGTHFLAPGTPARFSGSLLGGKAALT LSGVQPEDEAEYYCALWYSNRWVFGGGTKLTVL |
| 41 | 78-P3-1-scFv VH | EVQLVESGGGLVQPGGSLKLSCAASGFTFSKYAMNWV RQASGKGLEWVGRIRSKYNNYETYYADSVKDRFTISRD DSKNTAYLQMNNLKTEDTAVYYCVRHGNFGNSYISYW AYWGQGTTVTVSS |
| 42 | 79-P1-3-scFv VL | QTVVTQEPSLTVSPGGTVTLTCGSSTGAVTSGYYPNWV QQKPGQAPRALIYGTKFLAPGTPARFSGSLLGGKAALT LSGVQPEDEAEYYCALWQENRWVFGGGTKLTVL |
| 43 | 79-P2-3-scFv VL | QTVVTQEPSLTVSPGGTVTLTCGSSTGAVTSGYYPNWV QQKPGQAPRALIGGTKFLAPGTPARFSGSLLGGKAALT LSGVQPEDEAEYYCALWQENRWVFGGGTKLTVL |
| 44 | 79-P3-1-scFv VL | QTVVTQEPSLTVSPGGTVTLTCGSSTGAVTSGYYPNWV QQKPGQAPRGLIGGTKFLAPGTPARFSGSLLGGKAALT LSGVQPEDEAEYYCALWQENRWVFGGGTKLTVL |
| 45 | H2C, P1-3-scFv, 63-scFv, 79-scFv, 79-scFv HCDR1 | GFTFNKYAMN |
| 46 | H2C, P1-3-scFv, P2-3-scFv, P3-1-scFv, 63-scFv, 79-scFv, P1-3-3-scFv, P2-3-3-scFv, P2-3-5-scFv, 63-P2-3-scFv, 63-P3-1-scFv, 79-P1-3-scFv, 79-P2-3-scFv, 79-P3-1-scFv HCDR2 | RIRSKYNNYATY |
| 47 | H2C, P1-3-scFv, P2-3-scFv, P3-1-scFv, 63-scFv, 78-scFv, 79-scFv, P1-3-3-scFv, P2-3-3-scFv, P2-3-5-scFv, 63-P2-3-scFv, 63-P3-1-scFv, 78-P1-3-scFv, 78-P2-3-scFv, 78-P3-1-scFv, 79-P1-3-scFv, 79-P2-3-scFv, 79-P3-1-scFv HCDR3 | HGNFGNSYISYWAY |
| 48 | H2C, P1-3-scFv, P2-3-scFv, P3-1-scFv, 63-scFv, 78-scFv, 79-scFv, P1-3-3-scFv, P2-3-3-scFv, P2-3-5-scFv, 63-P2-3-scFv, 63-P3-1-scFv, 78-P1-3-scFv, 78-P2-3-scFv, 78-P3-1-scFv, 79-P1-3-scFv, 79-P2-3-scFv, 79-P3-1-scFv LCDR1 | GSSTGAVTSGYYPN |
| 49 | H2C, P1-3-scFv, P2-3-scFv, P3-1-scFv, 63-scFv, 79-scFv, P1-3-3-scFv, P2-3-3-scFv, P2-3-5-scFv, 63-P2-3-scFv, 63-P3-1-scFv, 79-P1-3-scFv, 79-P2-3-scFv, 79-P3-1-scFv LCDR2 | GTKFLAP |
| 50 | H2C, P1-3-scFv, P2-3-scFv, P3-1-scFv, 78-scFv, P1-3-3-scFv, P2-3-3-scFv, P2-3-5-scFv, 78-P1-3-scFv, 78-P2-3-scFv, 78-P3-1-scFv LCDR3 | ALWYSNRWV |
| 51 | P2-3-scFv, P3-1-scFv, P1-3-3-scFv, P2-3-3-scFv, P2-3-5-scFv, 63-P2-3-scFv, 63-P3-1-scFv, 78-P2-3-scFv, 78-P3-1-scFv, 79-P2-3-scFv, 79-P3-1-scFv HCDR1 | GFTFSKYAMN |
| 52 | 78-scFv, 78-P1-3-scFv HCDR1 | GFTFPKYAMN |
| 53 | 78-scFv, 78-P1-3-scFv, 78-P2-3-scFv, 78-P3-1-scFv HCDR2 | RIRSKYNNYETY |
| 54 | 78-scFv, 78-P1-3-scFv, 78-P2-3-scFv, 78-P3-1-scFv LCDR2 | GTHFLAP |
| 55 | 63-scFv, 63-P2-3-scFv, 63-P3-1-scFv, LCDR3 | ALWVDNRWV |
| 56 | 79-scFv, 79-P1-3-scFv, 79-P2-3-scFv, 79-P3-1-scFv LCDR3 | ALWQENRWV |
| 57 | H2C, P1-3-scFv, P2-3-scFv, P3-1-scFv, 63-scFv, 78-scFv, 79-scFv, P1-3-3-scFv, P2-3-3-scFv, P2-3-5-scFv, 63-P2-3-scFv, 63-P3-1-scFv, 78-P1-3-scFv, 78-P2-3-scFv, 78-P3-1-scFv, 79-P1-3-scFv, 79-P2-3-scFv, 79-P3-1-scFv HFR1 | EVQLVESGGGLVQPGGSLKLSCAAS |
| 58 | H2C, P2-3-scFv, 63-scFv, 78-scFv, 79-scFv 63-P2-3-scFv, 78-P2-3-scFv, 79-P2-3-scFv HFR2 | WVRQAPGKGLEWVA |
| 59 | H2C, P1-3-scFv, P2-3-scFv, P3-1-scFv, 63-scFv, 78-scFv, 79-scFv, P1-3-3-scFv, P2-3-3-scFv, P2-3-5-scFv, 63-P2-3-scFv, 63-P3-1-scFv, 78-P1-3-scFv, 78-P2-3-scFv, 78-P3-1-scFv, 79-P1-3-scFv, 79-P2-3-scFv, 79-P3-1-scFv HFR3 | YADSVKDRFTISRDDSKNTAYLQMNNLKTEDTAVYYC VR |
| 60 | H2C, 63-scFv, 78-scFv, 79-scFv HFR4 | WGQGTLVTVSS |
| 61 | H2C, P1-3-scFv, P2-3-scFv, P3-1-scFv, 63-scFv, 78-scFv, 79, P1-3-3-scFv, P2-3-3-scFv, P2-3-5-scFv, 63-P2-3-scFv, 63-P3-1-scFv, 78-P1-3-scFv, 78-P2-3-scFv, 78-P3-1-scFv, 79-P1-3-scFv, 79-P2-3-scFv, 79-P3-1-scFv LFR1 | QTVVTQEPSLTVSPGGTVTLTC |
| 62 | H2C, P3-1-scFv, 63-scFv, 78-scFv, 79-scFv, 63-P3-1-scFv, 78-P3-1-scFv, 79-P3-1-scFv LFR2 | WVQQKPGQAPRGLIG |
| 63 | H2C, P1-3-scFv, P2-3-scFv, P3-1-scFv, 63-scFv, 78-scFv, 79-scFv, P1-3-3-scFv, P2-3-3-scFv, P2-3-5-scFv, 63-P2-3-scFv, 63-P3-1-scFv, 78-P1-3-scFv, 78-P2-3-scFv, 78-P3-1-scFv, 79-P1-3-scFv, 79-P2-3-scFv, 79-P3-1-scFv LFR3 | GTPARFSGSLLGGKAALTLSGVQPEDEAEYYC |
| 64 | H2C, P1-3-scFv, P2-3-scFv, P3-1-scFv, 63-scFv, 78-scFv, 79-scFv, P1-3-3-scFv, P2-3-3-scFv, P2-3-5-scFv, 63-P2-3-scFv, 63-P3-1-scFv, 78-P1-3-scFv, 78-P2-3-scFv, 78-P3-1-scFv, 79-P1-3-scFv, 79-P2-3-scFv, 79-P3-1-scFv LFR4 | FGGGTKLTVL |
| 65 | P1-3-scFv, P2-3-3-scFv, 78-P1-3-scFv, 79-P1-3-scFv HFR2 | WVRQAPGKGLEWVG |
| 66 | P3-1-scFv, P1-3-3-scFv, P2-3-5-scFv, 63-P3-1-scFv, 78-P3-1-scFv, 79-P3-1-scFv HFR2 | WVRQASGKGLEWVG |
| 67 | P1-3-scFv, P2-3-scFv, P3-1-scFv, P1-3-3-scFv, P2-3-3-scFv, P2-3-5-scFv, 63-P2-3-scFv, 63-P3-1-scFv, 78-P1-3-scFv, 78-P2-3-scFv, 78-P3-1-scFv, 79-P1-3-scFv, 79-P2-3-scFv, 79-P3-1-scFv HFR4 | WGQGTTVTVSS |
| 68 | P1-3-scFv, P1-3-3-scFv, 78-P1-3-scFv, 79-P1-3-scFv LFR2 | WVQQKPGQAPRALIY |
| 69 | P2-3-scFv, P2-3-3-scFv, P2-3-5-scFv, 63-P2-3-scFv, 78-P2-3-scFv, 79-P2-3-scFv LFR2 | WVQQKPGQAPRALIG |
| 70 | Linking peptide | GGGGSGGGGSGGGGS |
| 71 | P1-1-scFv | QTVVTQEPSLTVSPGGTVTLTCGSSTGAVTSGYYPNWV QQKPGQAPRGLIGGTKFLAPGTPARFSGSLLGGKAALT LSGVQPEDEAEYYCALWYSNRWVFGGGTKLTVLGGG GSGGGGSGGGGSEVQLVESGGGLVQPGGSLKLSCAAS GFTFSKYAMNWVRQAPGKGLEWVARIRSKYNNYATY YADSVKDRFTISRDDSKNTAYLQMNSLKTEDTAVYYC VRHGNFGNSYISYWAYWGQGTTVTVSS |
| 72 | P2-1-scFv | QTVVTQEPSLTVSPGGTVTLTCGSSTGAVTSGYYPNWV QQKPGQAPRGLIGGTKFLAPGTPARFSGSLLGGKAALT LSGVQPEDEAEYYCALWYSNRWVFGGGTKLTVLGGG GSGGGGSGGGGSEVQLVESGGGLVQPGGSLKLSCAAS GFTFNKYAINWVRQAPGKGLEWVGRIRSKYNNYATYY ADSVKDRFTISRDDSKNTAYLQMNSLKTEDTAVYYCTR HGNFGNSYISYWAYWGQGTTVTVSS |
| 73 | P2-2-scFv | QTVVTQEPSLTVSPGGTVTLTCGSSTGAVTSGYYPNWV QQKPGQAPRALIGGTKFLAPGTPARFSGSLLGGKAALT LSGVQPEDEAEYYCALWYSNRWVFGGGTKLTVLGGG |
| | | GSGGGGSGGGGSEVQLVESGGGLVQPGGSLKLSCAAS GFTFSKYAMNWVRQAPGKGLEWVARIRSKYNNYATY YADSVKDRFTISRDDSKNTAYLQMNNLKTEDTAVYYC TRHGNFGNSYISYWAYWGQGTTVTVSS |
| 74 | P3-4-scFv | QTVVTQEPSLTVSPGGTVTLTCGSSTGAVTSGYYPNWV QQKPGQAPRALIGGTKFLAPGTPARFSGSLLGGKAALT LSGVQPEDEAEYYCALWYSNRWVFGGGTKLTVLGGG GSGGGGSGGGGSEVQLVESGGGLVQPGGSLKLSCAAS GFTFNKYAMNWVRQAPGKGLEWVARIRSKYNNYATY YADSVKDRFTISRDDSKNTAYLQMNSLKTEDTAVYYC VRHGNFGNSYISYWAYWGQGTTVTVSS |
| 75 | 16-scFv | QTVVTQEPSLTVSPGGTVTLTCGSSTGAVTSGYYPNWV QQKPGQAPRGLIGGTKFLAPGTPARFSGSLLGGKAALT LSGVQPEDEAEYYCALWYSDTWVFGGGTKLTVLGGG GSGGGGSGGGGSEVQLVESGGGLVQPGGSLKLSCAAS GFTFNKYAMNWVRQAPGKGLEWVARIRSKYNNYATY YADSVKDRFTISRDDSKNTAYLQMNNLKTEDTAVYYC VRHGNFGNSYISYWAYWGQGTLVTVSS |
| 76 | 23-scFv | QTVVTQEPSLTVSPGGTVTLTCGSSTGAVTSGYYPNWV QQKPGQAPRGLIGGTKFLAPGTPARFSGSLLGGKAALT LSGVQPEDEAEYYCALWLANRWVFGGGTKLTVLGGG GSGGGGSGGGGSEVQLVESGGGLVQPGGSLKLSCAAS GFTFNKYAMNWVRQAPGKGLEWVARIRSKYNNYATY YADSVKDRFTISRDDSKNTAYLQMNNLKTEDTAVYYC VRHGNFGNSYISYWAYWGQGTLVTVSS |
| 77 | 34-scFv | QTVVTQEPSLTVSPGGTVTLTCGSSTGTVTSGYYPNWV QQKPGQAPRGLIGGTKFLAPGTPARFSGSLLGGKAALT LSGVQPEDEAEYYCALWLSNRWVFGGGTKLTVLGGGG SGGGGSGGGGSEVQLVESGGGLVQPGGSLKLSCAASGF TFNKYAMNWVRQAPGKGLEWVARIRSKYNNYATYYA DSVKDRFTISRDDSKNTAYLQMNNLKTEDTAVYYCVR HGNFGNSGISYWAYWGQGTLVTVSS |
| 78 | 43-scFv | QTVVTQEPSLTVSPGGTVTLTCGSSTGAVTSGYYPNWV QQKPGQAPRGLIGGTKFLAPGTPARFSGSLLGGKAALT LSGVQPEDEAEYYCALWYSNRWVFGGGTKLTVLGGG GSGGGGSGGGGSEVQLVESGGGLVQPGGSLKLSCAAS GFTFNKYAMNWVRQAPGKGLEWVARIRSKYNNYATY |
| | | YADSVKDRFTISRDDSKNTAYLQMNNLKTEDTAVYYC VRHGNFGTSYISYWAYWGQGTLVTVSS |
| 79 | 67-scFv | QTVVTQEPSLTVSPGGTVTLTCGSSTGAVTSGYYPNWV QQKPGQAPRGLIGGTKFLAPGTPARFSGSLLGGKAALT LSGVQPEDEAEYYCALWYSNRWVFGGGTKLTVLGGG GSGGGGSGGGGSEVQLVESGGGLVQPGGSLKLSCAAS GFTFNKYAMNWVRQAPGKGLEWVARIRSKYNNYATY YADSVKDRFTISRDDSKNTAYLQMNNLKTEDTAVYYC VRHGNFGNTGISYWAYWGQGTLVTVSS |
| 80 | 73-scFv | QTVVTQEPSLTVSPGGTVTLTCGSSTGAVTSGYYPNWV QQKPGQAPRGLIGGTKFLAPGTPARFSGSLLGGKAALT LSGVQPEDEAEYYCALWYSGQWVFGGGTKLTVLGGG GSGGGGSGGGGSEVQLVESGGGLVQPGGSLKLSCAAS GFTFNKYAMNWVRQAPGKGLEWVARIRSKYNNYATY YADSVKDRFTISRDDSKNTAYLQMNNLKTEDTAVYYC VRHGNFGNSYISYWAYWGQGTLVTVSS |
| 81 | Monkey CD3E | MQSGTRWRVLGLCLLSIGVWGQDGNEEMGSITQTPYQ VSISGTTVILTCSQHLGSEAQWQHNGKNKEDSGDRLFL PEFSEMEQSGYYVCYPRGSNPEDASHHLYLKARVCENC MEMDVMAVATIVIVDICITLGLLLLVYYWSKNRKAKA KPVTRGAGAGGRQRGQNKERPPPVPNPDYEPIRKGQQD LYSGLNQRRI |

## Claims

1. An antibody or antigen-binding fragment specifically binding to CD3, **characterized in that** the antibody or antigen-binding fragment specifically binding to CD3 comprises
(a) 3 CDRs in a heavy chain variable region amino acid sequence as set forth in SEQ ID NO: 28 and 3 CDRs in a light chain variable region amino acid sequence as set forth in SEQ ID NO: 29;
(b) 3 CDRs in a heavy chain variable region amino acid sequence as set forth in SEQ ID NO: 1 and 3 CDRs in a light chain variable region amino acid sequence as set forth in SEQ ID NO: 31;
(c) 3 CDRs in a heavy chain variable region amino acid sequence as set forth in SEQ ID NO: 32 and 3 CDRs in a light chain variable region amino acid sequence as set forth in SEQ ID NO: 33;
(d) 3 CDRs in a heavy chain variable region amino acid sequence as set forth in SEQ ID NO: 1 and 3 CDRs in a light chain variable region amino acid sequence as set forth in SEQ ID NO: 34;
(e) 3 CDRs in a heavy chain variable region amino acid sequence as set forth in SEQ ID NO: 28 and 3 CDRs in a light chain variable region amino acid sequence as set forth in SEQ ID NO: 36;
(f) 3 CDRs in a heavy chain variable region amino acid sequence as set forth in SEQ ID NO: 39 and 3 CDRs in a light chain variable region amino acid sequence as set forth in SEQ ID NO: 40; or
(g) 3 CDRs in a heavy chain variable region amino acid sequence as set forth in SEQ ID NO: 28 and 3 CDRs in a light chain variable region amino acid sequence as set forth in SEQ ID NO: 43.

2. An antibody or antigen-binding fragment specifically binding to CD3, **characterized in that** the antibody or antigen-binding fragment specifically binding to CD3 comprises a heavy chain variable region and a light chain variable region, wherein
(a) the heavy chain variable region comprises, according to AbM numbering, HCDR1 as set forth in GFTFSKYAMN (SEQ ID NO: 51), HCDR2 as set forth in RIRSKYNNYATY (SEQ ID NO: 46), and HCDR3 as set forth in HGNFGNSYISYWAY (SEQ ID NO: 47); the light chain variable region comprises, according to AbM numbering, LCDR1 as set forth in GSSTGAVTSGYYPN (SEQ ID NO: 48), LCDR2 as set forth in GTKFLAP (SEQ ID NO: 49), and LCDR3 as set forth in ALWYSNRWV (SEQ ID NO: 50);
(b) the heavy chain variable region comprises, according to AbM numbering, HCDR1 as set forth in GFTFNKYAMN (SEQ ID NO: 45), HCDR2 as set forth in RIRSKYNNYATY (SEQ ID NO: 46), and HCDR3 as set forth in HGNFGNSYISYWAY (SEQ ID NO: 47); the light chain variable region comprises, according to AbM numbering, LCDR1 as set forth in GSSTGAVTSGYYPN (SEQ ID NO: 48), LCDR2 as set forth in GTKFLAP (SEQ ID NO: 49), and LCDR3 as set forth in ALWVDNRWV (SEQ ID NO: 55);
(c) the heavy chain variable region comprises, according to AbM numbering, HCDR1 as set forth in GFTFPKYAMN (SEQ ID NO: 52), HCDR2 as set forth in RIRSKYNNYETY (SEQ ID NO: 53), and HCDR3 as set forth in HGNFGNSYISYWAY (SEQ ID NO: 47); the light chain variable region comprises, according to AbM numbering, LCDR1 as set forth in GSSTGAVTSGYYPN (SEQ ID NO: 48), LCDR2 as set forth in GTHFLAP (SEQ ID NO: 54), and LCDR3 as set forth in ALWYSNRWV (SEQ ID NO: 50);
(d) the heavy chain variable region comprises, according to AbM numbering, HCDR1 as set forth in GFTFNKYAMN (SEQ ID NO: 45), HCDR2 as set forth in RIRSKYNNYATY (SEQ ID NO: 46), and HCDR3 as set forth in HGNFGNSYISYWAY (SEQ ID NO: 47); the light chain variable region comprises, according to AbM numbering, LCDR1 as set forth in GSSTGAVTSGYYPN (SEQ ID NO: 48), LCDR2 as set forth in GTKFLAP (SEQ ID NO: 49), and LCDR3 as set forth in ALWQENRWV (SEQ ID NO: 56);
(e) the heavy chain variable region comprises, according to AbM numbering, HCDR1 as set forth in GFTFSKYAMN (SEQ ID NO: 51), HCDR2 as set forth in RIRSKYNNYATY (SEQ ID NO: 46), and HCDR3 as set forth in HGNFGNSYISYWAY (SEQ ID NO: 47); the light chain variable region comprises, according to AbM numbering, LCDR1 as set forth in GSSTGAVTSGYYPN (SEQ ID NO: 48), LCDR2 as set forth in GTKFLAP (SEQ ID NO: 49), and LCDR3 as set forth in ALWVDNRWV (SEQ ID NO: 55);
(f) the heavy chain variable region comprises, according to AbM numbering, HCDR1 as set forth in GFTFSKYAMN (SEQ ID NO: 51), HCDR2 as set forth in RIRSKYNNYETY (SEQ ID NO: 53), and HCDR3 as set forth in HGNFGNSYISYWAY (SEQ ID NO: 47); the light chain variable region comprises, according to AbM numbering, LCDR1 as set forth in GSSTGAVTSGYYPN (SEQ ID NO: 48), LCDR2 as set forth in GTHFLAP (SEQ ID NO: 54), and LCDR3 as set forth in ALWYSNRWV (SEQ ID NO: 50); or
(g) the heavy chain variable region comprises, according to AbM numbering, HCDR1 as set forth in GFTFSKYAMN (SEQ ID NO: 51), HCDR2 as set forth in RIRSKYNNYATY (SEQ ID NO: 46), and HCDR3 as set forth in HGNFGNSYISYWAY (SEQ ID NO: 47); the light chain variable region comprises, according to AbM numbering, LCDR1 as set forth in GSSTGAVTSGYYPN (SEQ ID NO: 48), LCDR2 as set forth in GTKFLAP (SEQ ID NO: 49), and LCDR3 as set forth in ALWQENRWV (SEQ ID NO: 56).

3. The antibody or antigen-binding fragment specifically binding to CD3 according to claim 2, **characterized in that** the antibody or antigen-binding fragment specifically binding to CD3 comprises
(a) a heavy chain variable region as set forth in SEQ ID NO: 26 and a light chain variable region as set forth in SEQ ID NO: 27;
(b) a heavy chain variable region as set forth in SEQ ID NO: 28 and a light chain variable region as set forth in SEQ ID NO: 29;
(c) a heavy chain variable region as set forth in SEQ ID NO: 30 and a light chain variable region as set forth in SEQ ID NO: 2;
(d) a heavy chain variable region as set forth in SEQ ID NO: 1 and a light chain variable region as set forth in SEQ ID NO: 31;
(e) a heavy chain variable region as set forth in SEQ ID NO: 32 and a light chain variable region as set forth in SEQ ID NO: 33;
(f) a heavy chain variable region as set forth in SEQ ID NO: 1 and a light chain variable region as set forth in SEQ ID NO: 34;
(g) a heavy chain variable region as set forth in SEQ ID NO: 30 and a light chain variable region as set forth in SEQ ID NO: 27;
(h) a heavy chain variable region as set forth in SEQ ID NO: 35 and a light chain variable region as set forth in SEQ ID NO: 29;
(i) a heavy chain variable region as set forth in SEQ ID NO: 30 and a light chain variable region as set forth in SEQ ID NO: 29;
(j) a heavy chain variable region as set forth in SEQ ID NO: 28 and a light chain variable region as set forth in SEQ ID NO: 36;
(k) a heavy chain variable region as set forth in SEQ ID NO: 30 and a light chain variable region as set forth in SEQ ID NO: 31;
(l) a heavy chain variable region as set forth in SEQ ID NO: 37 and a light chain variable region as set forth in SEQ ID NO: 38;
(m) a heavy chain variable region as set forth in SEQ ID NO: 39 and a light chain variable region as set forth in SEQ ID NO: 40;
(n) a heavy chain variable region as set forth in SEQ ID NO: 41 and a light chain variable region as set forth in SEQ ID NO: 33;
(o) a heavy chain variable region as set forth in SEQ ID NO: 26 and a light chain variable region as set forth in SEQ ID NO: 42;
(p) a heavy chain variable region as set forth in SEQ ID NO: 28 and a light chain variable region as set forth in SEQ ID NO: 43; or
(q) a heavy chain variable region as set forth in SEQ ID NO: 30 and a light chain variable region as set forth in SEQ ID NO: 44.

4. The antibody or antigen-binding fragment specifically binding to CD3 according to any one of claims 1 to 3, **characterized in that** the antibody or antigen-binding fragment is an IgGl, IgG2, IgG3, or IgG4 antibody; optionally, an IgG1 or IgG4 antibody; optionally, an IgG1 antibody.

5. The antibody or antigen-binding fragment specifically binding to CD3 according to any one of claims 1 to 4, **characterized in that** the antigen-binding fragment is Fab, Fab', F(ab')2, Fv, single chain Fv, single chain Fab or diabody.

6. The antibody or antigen-binding fragment specifically binding to CD3 according to any one of claims 1 to 5, **characterized in that** the antibody or antigen-binding fragment specifically binding to CD3 has one or more of the following properties:
(i) a monomer purity greater than 90%, preferably greater than 92%, 94%, 96%, or 97%, as measured by SEC-HPLC;
(ii) a purity greater than 90%, preferably greater than 91%, 92%, or 93%, as measured by reducing or non-reducing SDS-PAGE; or
(iii) specifically binding to CD3 expressed on a cell surface.

7. An isolated nucleic acid, **characterized in that** the isolated nucleic acid encodes the anti-CD3 antibody or the antigen-binding fragment thereof according to any one of claims 1 to 6.

8. A vector, **characterized in that** the vector comprises the nucleic acid according to claim 7, preferably the vector is an expression vector.

9. A host cell, **characterized in that** the host cell comprises the nucleic acid according to claim 7 or the vector according to claim 8, preferably the host cell is prokaryotic or eukaryotic, more preferably the host cell is selected from an E. coli cell, a yeast cell, a mammalian cell or other cells suitable for the preparation of an antibody or an antigen-binding fragment thereof, and most preferably the host cell is a 293 cell or a CHO cell.

10. A method for preparing the anti-CD3 antibody or the antigen-binding fragment thereof according to any one of claims 1 to 6, **characterized in that** the method comprises culturing the host cell according to claim 9 under conditions suitable for the expression of a nucleic acid encoding the anti-CD3 antibody or the antigen-binding fragment thereof according to any one of claims 1 to 6, and optionally the method further comprises recovering the anti-CD3 antibody or the antigen-binding fragment thereof from a culture of the host cell.

11. A pharmaceutical composition, **characterized in that** the pharmaceutical composition comprises the anti-CD3 antibody or the antigen-binding fragment thereof according to any one of claims 1 to 6, and optionally a pharmaceutically acceptable carrier.

12. The anti-CD3 antibody or the antigen-binding fragment thereof according to any one of claims 1 to 6 or the pharmaceutical composition according to claim 11 for use in preventing or treating cancer, an infectious disease, or an autoimmune disease in a subject.
